(19) Europäisches Patentamt
European Patent Office
Office européen des brevets

(11) **EP 4 778 539 A1**

(12) **EUROPEAN PATENT APPLICATION**
published in accordance with Art. 153(4) EPC

(43) Date of publication:
**22.07.2026 Bulletin 2026/30**

(21) Application number: 24865500.3

(22) Date of filing: **12.09.2024**

(51) International Patent Classification (IPC):
*A61K 47/68* (2017.01)     *A61K 39/395* (2006.01)
*A61K 51/10* (2006.01)     *A61P 35/00* (2006.01)
*A61P 35/02* (2006.01)     *C07K 16/18* (2006.01)

(52) Cooperative Patent Classification (CPC):
A61K 39/395; A61K 47/68; A61K 51/10;
A61P 35/00; A61P 35/02; C07K 16/18

(86) International application number:
**PCT/JP2024/032627**

(87) International publication number:
**WO 2025/058000 (20.03.2025 Gazette 2025/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.09.2023 JP 2023148229**

(71) Applicant: **Toray Industries, Inc.**
**Tokyo 103-8666 (JP)**

(72) Inventors:
• **MAJIMA, Yuki**
  **Kamakura-shi, Kanagawa 248-8555 (JP)**
• **OKANO, Fumiyoshi**
  **Kamakura-shi, Kanagawa 248-8555 (JP)**

(74) Representative: **Mewburn Ellis LLP**
**Aurora Building**
**Counterslip**
**Bristol BS1 6BX (GB)**

Remarks:
The complete document including Reference
Table(s) and the Sequence Listing(s) can be
downloaded from the EPO website

(54) **PHARMACEUTICAL COMPOSITION FOR TREATING, PREVENTING AND/OR DIAGNOSING CANCER**

(57)     A complex, in which a radioisotope(s) and an antibody or a fragment thereof having immunological reactivity with a CAPRIN-1 protein having an amino acid sequence shown in any one of the even numbered SEQ ID NOs: 2 to 30, or an amino acid sequence having 80% or more sequence identity with the amino acid sequence are bound via a chelating agent(s), has an excellent anti-tumor effect and an excellent cancer detection capability. Therefore, the complex can be utilized as a radiopharmaceutical.

**EP 4 778 539 A1**

**Description**

Technical Field

The present invention relates to a complex in which an antibody against a CAPRIN-1 protein or a fragment thereof is labeled with a radioisotope; and a pharmaceutical composition for treatment, prevention, and/or diagnosis of a cancer, using the complex.

Background

Various antibody medicines targeting specific antigen proteins on cancer cell surfaces are applied as therapeutic agents for cancers with fewer side effects as a cancer treatment because of their cancer specificity. For example, cytoplasmic-activation and proliferation-associated protein 1 (CAPRIN-1) is specifically expressed on cell membrane surfaces of many solid cancers. Antibodies against this CAPRIN-1 protein are known to be promising in pharmaceutical uses for treatment and/or prevention of cancers (Patent Literature 1).
In recent years, for pharmaceuticals for treatment, prevention, and/or diagnosis of cancers, complexes, in which unsealed radioisotopes and molecules targeting cancer tissues or cancer cells are bound, have been practically used as radiopharmaceuticals. Administration of such a radiopharmaceutical to a cancer patient allows the radiopharmaceutical to accumulate in a cancer tissue or a cancer cell, and enables treatment or detection of a cancer due to radiation emitted from the radioisotope (Non Patent Literatures 1 and 2).
Antibodies specifically having immunological reactivity with the surfaces of the cancer tissues or the cancer cells can be utilized as the molecules targeting the cancer tissues or the cancer cells. For example, a therapeutic agent (Zevalin (registered trademark)), in which a monoclonal antibody against CD20 is labeled with indium-111 (111-In) or yttrium-90 (Y-90) which is one of radioisotopes, has been practically used for treatment and diagnosis of CD20-positive recurrent or refractory low-grade B-cell non-Hodgkin's lymphoma and mantle cell lymphoma.
Ligands for receptors expressed on the surfaces of the cancer tissues or the cancer cells can be used as the molecules targeting the cancer tissues or the cancer cells. Examples thereof include a diagnostic drug for cancer (Octreoscan (registered trademark)), which is targeted for a somatostatin receptor-positive neuroendocrine tumor, and in which a somatostatin analog is labeled with indium-111 (111-In), a therapeutic agent for cancer (Lutathera (registered trademark)), in which a somatostatin analog is labeled with lutetium 177 (Lu -177), and vipivotide tetraxetan (PLUVICTO (registered trademark)) comprising PMSA analog lutetium 177 (Lu-177), for treatment of PMSA-positive metastatic castration-resistant prostate cancer (mCRPC) in a subject having a history of treatment with an androgen receptor (AR) pathway blocking agent and a taxane-based anticancer agent.

Citation List

Patent Literature

[Patent Literature 1]: WO2010/016526

Non Patent Literature

[Non Patent Literature 1]: J. Nucl. Med., 46, 67S-75S (2005)
[Non Patent Literature 2]: J. Nucl. Med., 46 (Suppl.1), 38S-47S (2005)

Summary

Technical Problem

Radiopharmaceuticals utilizing molecules targeting cancer tissues or cancer cells have already been practically used, as mentioned above. However, the radiopharmaceuticals have had limited anti-tumor effects. Thus, an objective of the present invention is to provide a radiopharmaceutical having an anti-tumor effect and a cancer detection capability superior to those of the conventional technology.

Solution to Problem

As a result of intensive studies, the present inventors have found that a complex, in which a radioisotope(s) is bound to an antibody having immunological reactivity with a CAPRIN-1 protein or a fragment thereof via a chelating agent(s), exhibits greater efficacy in the treatment, prevention, and/or diagnosis of a cancer compared to those of existing radiopharmaceuticals. On the basis of these, the present invention has been completed.

Specifically, the present invention has the following features (1) to (18).

(1) A complex, wherein a radioisotope(s) and an antibody or a fragment thereof having immunological reactivity with a CAPRIN-1 protein having an amino acid sequence shown in any one of the even numbered SEQ ID NOs: 2 to 30, or an amino acid sequence having 80% or more sequence identity with the amino acid sequence are bound via a chelating agent(s).

(2) The complex according to (1), wherein the antibody or the fragment thereof has immunological reactivity with a partial polypeptide of the CAPRIN-1 protein, and wherein the partial polypeptide has an amino acid sequence shown in any one of SEQ ID NOs: 31 to 35, 296 to 299, 308, and 309, or an amino acid sequence having 80% or more sequence identity with the amino acid sequence.

(3) The complex according to (1) or (2), wherein the antibody is a monoclonal antibody or a polyclonal antibody.

(4) The complex according to any one of (1) to (3), wherein the antibody or the fragment thereof is any one of the following (A) to (M):

(A) an antibody or a fragment thereof comprising a heavy-chain variable region comprising complementarity determining regions of SEQ ID NOs: 36, 37, and 38 (CDR1, CDR2, and CDR3, respectively) and a light-chain variable region comprising complementarity determining regions of SEQ ID NOs: 40, 41, and 42 (CDR1, CDR2, and CDR3, respectively), and having an immunological reactivity with the CAPRIN-1 protein;

(B) an antibody or a fragment thereof comprising a heavy-chain variable region comprising complementarity determining regions of SEQ ID NOs: 44, 45, and 46 (CDR1, CDR2, and CDR3, respectively) and a light-chain variable region comprising complementarity determining regions of SEQ ID NOs: 48, 49, and 50 (CDR1, CDR2, and CDR3, respectively), and having an immunological reactivity with the CAPRIN-1 protein;

(C) an antibody or a fragment thereof comprising a heavy-chain variable region comprising complementarity determining regions of SEQ ID NOs: 52, 53, and 54 (CDR1, CDR2, and CDR3, respectively) and a light-chain variable region comprising complementarity determining regions of SEQ ID NOs: 56, 57, and 58 (CDR1, CDR2, and CDR3, respectively), and having an immunological reactivity with the CAPRIN-1 protein;

(D) an antibody or a fragment thereof comprising a heavy-chain variable region comprising complementarity determining regions of SEQ ID NOs: 60, 61, and 62 (CDR1, CDR2, and CDR3, respectively) and a light-chain variable region comprising complementarity determining regions of SEQ ID NOs: 64, 65, and 66 (CDR1, CDR2, and CDR3, respectively), and having an immunological reactivity with the CAPRIN-1 protein;

(E) an antibody or a fragment thereof comprising a heavy-chain variable region comprising complementarity determining regions of SEQ ID NOs: 170, 171, and 172 (CDR1, CDR2, and CDR3, respectively) and a light-chain variable region comprising complementarity determining regions of SEQ ID NOs: 173, 174, and 175 (CDR1, CDR2, and CDR3, respectively), and having an immunological reactivity with the CAPRIN-1 protein;

(F) an antibody or a fragment thereof comprising a heavy-chain variable region comprising complementarity determining regions of SEQ ID NOs: 176, 177, and 178 (CDR1, CDR2, and CDR3, respectively) and a light-chain variable region comprising complementarity determining regions of SEQ ID NOs: 179, 180, and 181 (CDR1, CDR2, and CDR3, respectively), and having an immunological reactivity with the CAPRIN-1 protein;

(G) an antibody or a fragment thereof comprising a heavy-chain variable region comprising complementarity determining regions of SEQ ID NOs: 182, 183, and 184 (CDR1, CDR2, and CDR3, respectively) and a light-chain variable region comprising complementarity determining regions of SEQ ID NOs: 185, 186, and 187 (CDR1, CDR2, and CDR3, respectively), and having an immunological reactivity with the CAPRIN-1 protein;

(H) an antibody or a fragment thereof comprising a heavy-chain variable region comprising complementarity determining regions of SEQ ID NOs: 188, 189, and 190 (CDR1, CDR2, and CDR3, respectively) and a light-chain variable region comprising complementarity determining regions of SEQ ID NOs: 191, 192, and 193 (CDR1, CDR2, and CDR3, respectively), and having an immunological reactivity with the CAPRIN-1 protein;

(I) an antibody or a fragment thereof comprising a heavy-chain variable region comprising complementarity determining regions of SEQ ID NOs: 146, 147, and 148 (CDR1, CDR2, and CDR3, respectively) and a light-chain variable region comprising complementarity determining regions of SEQ ID NOs: 149, 150, and 151 (CDR1, CDR2, and CDR3, respectively), and having an immunological reactivity with the CAPRIN-1 protein;

(J) an antibody or a fragment thereof comprising a heavy-chain variable region comprising complementarity determining regions of SEQ ID NOs: 272, 273, and 274 (CDR1, CDR2, and CDR3, respectively) and a light-chain variable region comprising complementarity determining regions of SEQ ID NOs: 275, 276, and 277 (CDR1, CDR2, and CDR3, respectively), and having an immunological reactivity with the CAPRIN-1 protein;

(K) an antibody or a fragment thereof comprising a heavy-chain variable region comprising complementarity determining regions of SEQ ID NOs: 290, 291, and 292 (CDR1, CDR2, and CDR3, respectively) and a light-chain variable region comprising complementarity determining regions of SEQ ID NOs: 293, 294, and 295 (CDR1, CDR2, and CDR3, respectively), and having an immunological reactivity with the CAPRIN-1 protein;

(L) an antibody or a fragment thereof comprising a heavy-chain variable region comprising complementarity determining regions of SEQ ID NOs: 301, 302, and 303 (CDR1, CDR2, and CDR3, respectively) and a light-chain variable region comprising complementarity determining regions of SEQ ID NOs: 305, 306, and 307 (CDR1, CDR2, and CDR3, respectively), and having an immunological reactivity with the CAPRIN-1 protein; and

(M) an antibody or a fragment thereof comprising a heavy-chain variable region comprising complementarity determining regions of SEQ ID NOs: 134, 135, and 136 (CDR1, CDR2, and CDR3, respectively) and a light-chain variable region comprising complementarity determining regions of SEQ ID NOs: 137, 138, and 139 (CDR1, CDR2, and CDR3, respectively), and having an immunological reactivity with the CAPRIN-1 protein.

(5) The complex according to any one of (1) to (4), wherein the antibody or the fragment thereof is any one of the following (a) to (al):

(a) an antibody or a fragment thereof comprising a heavy-chain variable region comprising the amino acid sequence of SEQ ID NO: 39 and a light-chain variable region comprising the amino acid sequence of SEQ ID NO: 43;

(b) an antibody or a fragment thereof comprising a heavy-chain variable region comprising the amino acid sequence of SEQ ID NO: 47 and a light-chain variable region comprising the amino acid sequence of SEQ ID NO: 51;

(c) an antibody or a fragment thereof comprising a heavy-chain variable region comprising the amino acid sequence of SEQ ID NO: 55 and a light-chain variable region comprising the amino acid sequence of SEQ ID NO: 59;

(d) an antibody or a fragment thereof comprising a heavy-chain variable region comprising the amino acid sequence of SEQ ID NO: 63 and a light-chain variable region comprising the amino acid sequence of SEQ ID NO: 67;

(e) an antibody or a fragment thereof comprising a heavy-chain variable region comprising the amino acid sequence of SEQ ID NO: 68 and a light-chain variable region comprising the amino acid sequence of SEQ ID NO: 69;

(f) an antibody or a fragment thereof comprising a heavy-chain variable region comprising the amino acid sequence of SEQ ID NO: 70 and a light-chain variable region comprising the amino acid sequence of SEQ ID NO: 71;

(g) an antibody or a fragment thereof comprising a heavy-chain variable region comprising the amino acid sequence of SEQ ID NO: 72 and a light-chain variable region comprising the amino acid sequence of SEQ ID NO: 73;

(h) an antibody or a fragment thereof comprising a heavy-chain variable region comprising the amino acid sequence of SEQ ID NO: 74 and a light-chain variable region comprising the amino acid sequence of SEQ ID NO: 75;

(i) an antibody or a fragment thereof comprising a heavy-chain variable region comprising the amino acid sequence of SEQ ID NO: 76 and a light-chain variable region comprising the amino acid sequence of SEQ ID NO: 77;

(j) an antibody or a fragment thereof comprising a heavy-chain variable region comprising the amino acid sequence of SEQ ID NO: 78 and a light-chain variable region comprising the amino acid sequence of SEQ ID NO: 79;

(k) an antibody or a fragment thereof comprising a heavy-chain variable region comprising the amino acid sequence of SEQ ID NO: 80 and a light-chain variable region comprising the amino acid sequence of SEQ ID NO: 81;

(l) an antibody or a fragment thereof comprising a heavy-chain variable region comprising the amino acid sequence of SEQ ID NO: 82 and a light-chain variable region comprising the amino acid sequence of SEQ ID NO: 83;

(m) an antibody or a fragment thereof comprising a heavy-chain variable region comprising the amino acid sequence of SEQ ID NO: 84 and a light-chain variable region comprising the amino acid sequence of SEQ ID NO: 85;

(n) an antibody or a fragment thereof comprising a heavy-chain variable region comprising the amino acid sequence of SEQ ID NO: 86 and a light-chain variable region comprising the amino acid sequence of SEQ ID NO: 87;

(o) an antibody or a fragment thereof comprising a heavy-chain variable region comprising the amino acid sequence of SEQ ID NO: 88 and a light-chain variable region comprising the amino acid sequence of SEQ ID NO: 89;

(p) an antibody or a fragment thereof comprising a heavy-chain variable region comprising the amino acid sequence of SEQ ID NO: 90 and a light-chain variable region comprising the amino acid sequence of SEQ ID NO: 91;

(q) an antibody or a fragment thereof comprising a heavy-chain variable region comprising the amino acid sequence of SEQ ID NO: 92 and a light-chain variable region comprising the amino acid sequence of SEQ ID NO: 93;

(r) an antibody or a fragment thereof comprising a heavy-chain variable region comprising the amino acid sequence of SEQ ID NO: 94 and a light-chain variable region comprising the amino acid sequence of SEQ ID NO: 95;

(s) an antibody or a fragment thereof comprising a heavy-chain variable region comprising the amino acid sequence of SEQ ID NO: 96 and a light-chain variable region comprising the amino acid sequence of SEQ ID NO: 97;

(t) an antibody or a fragment thereof comprising a heavy-chain variable region comprising the amino acid sequence of SEQ ID NO: 98 and a light-chain variable region comprising the amino acid sequence of SEQ ID NO: 99;

(u) an antibody or a fragment thereof comprising a heavy-chain variable region comprising the amino acid sequence of SEQ ID NO: 100 and a light-chain variable region comprising the amino acid sequence of SEQ ID NO: 101;

(v) an antibody or a fragment thereof comprising a heavy-chain variable region comprising the amino acid sequence of SEQ ID NO: 102 and a light-chain variable region comprising the amino acid sequence of SEQ ID NO: 103;

(w) an antibody or a fragment thereof comprising a heavy-chain variable region comprising the amino acid sequence of SEQ ID NO: 104 and a light-chain variable region comprising the amino acid sequence of SEQ ID NO: 105;

(x) an antibody or a fragment thereof comprising a heavy-chain variable region comprising the amino acid sequence of SEQ ID NO: 106 and a light-chain variable region comprising the amino acid sequence of SEQ ID NO: 107;

(y) an antibody or a fragment thereof comprising a heavy-chain variable region comprising the amino acid sequence of SEQ ID NO: 108 and a light-chain variable region comprising the amino acid sequence of SEQ ID NO: 109;

(z) an antibody or a fragment thereof comprising a heavy-chain variable region comprising the amino acid sequence of SEQ ID NO: 110 and a light-chain variable region comprising the amino acid sequence of SEQ ID NO: 111;

(aa) an antibody or a fragment thereof comprising a heavy-chain variable region comprising the amino acid sequence of SEQ ID NO: 112 and a light-chain variable region comprising the amino acid sequence of SEQ ID NO: 113;

(ab) an antibody or a fragment thereof comprising a heavy-chain variable region comprising the amino acid sequence of SEQ ID NO: 114 and a light-chain variable region comprising the amino acid sequence of SEQ ID NO: 115;

(ac) an antibody or a fragment thereof comprising a heavy-chain variable region comprising the amino acid sequence of SEQ ID NO: 116 and a light-chain variable region comprising the amino acid sequence of SEQ ID NO: 117;

(ad) an antibody or a fragment thereof comprising a heavy-chain variable region comprising the amino acid sequence of SEQ ID NO: 118 and a light-chain variable region comprising the amino acid sequence of SEQ ID NO: 119;

(ae) an antibody or a fragment thereof comprising a heavy-chain variable region comprising the amino acid sequence of SEQ ID NO: 120 and a light-chain variable region comprising the amino acid sequence of SEQ ID NO: 121;

(af) an antibody or a fragment thereof comprising a heavy-chain variable region comprising the amino acid sequence of SEQ ID NO: 122 and a light-chain variable region comprising the amino acid sequence of SEQ ID NO: 123;

(ag) an antibody or a fragment thereof comprising a heavy-chain variable region comprising the amino acid sequence of SEQ ID NO: 124 and a light-chain variable region comprising the amino acid sequence of SEQ ID NO: 125;

(ah) an antibody or a fragment thereof comprising a heavy-chain variable region comprising the amino acid sequence of SEQ ID NO: 126 and a light-chain variable region comprising the amino acid sequence of SEQ ID NO: 127;

(ai) an antibody or a fragment thereof comprising a heavy-chain variable region comprising the amino acid sequence of SEQ ID NO: 128 and a light-chain variable region comprising the amino acid sequence of SEQ ID NO: 129;

(aj) an antibody or a fragment thereof comprising a heavy-chain variable region comprising the amino acid sequence of SEQ ID NO: 130 and a light-chain variable region comprising the amino acid sequence of SEQ ID NO: 131;

(ak) an antibody or a fragment thereof comprising a heavy-chain variable region comprising the amino acid sequence of SEQ ID NO: 132 and a light-chain variable region comprising the amino acid sequence of SEQ ID NO: 133; and

(al) an antibody or a fragment thereof comprising a heavy-chain variable region comprising the amino acid sequence of SEQ ID NO: 300 and a light-chain variable region comprising the amino acid sequence of SEQ ID NO: 304.

(6) The complex according to any one of (1) to (5), wherein the antibody is a human antibody, a humanized antibody, or a chimeric antibody.

(7) The complex according to any one of (1) to (5), wherein the fragment is F(ab')$_2$, Fab', Fab, or scFv.

(8) The complex according to any one of (1) to (7), wherein the radioisotope is a radioisotope that radiates an $\alpha$ ray, a $\beta$ ray, or a $\gamma$ ray.

(9) The complex according to any one of (1) to (8), wherein the radioisotope is radium (Ra), thorium (Th), actinium (Ac), lutetium (Lu), indium (In), gallium (Ga), lead (Pb), copper (Cu), strontium (Sr), yttrium (Y), samarium (Sm), rhenium (Re), bismuth (Bi), technetium (Tc), or zirconium (Zr).

(10) The complex according to any one of (1) to (9), wherein the radioisotope is 223-Ra, 227-Th, 225-Ac, 177-Lu, 111-In, 67-Ga, 68-Ga, 212-Pb, 64-Cu, 67-Cu, 89-Sr, 90-Y, 153-Sm, 186-Re, 188-Re, 212-Bi, 213-Bi, 99m-Tc, or 89-Zr.

(11) The complex according to any one of (1) to (10), wherein the chelating agent is a bifunctional chelating agent.

(12) The complex according to (11), wherein the bifunctional chelating agent is a cyclic or linear chelating agent.

(13) The complex according to (12), wherein the cyclic chelating agent is selected from a group consisting of DOTA, NOTA, TETA, TCMC, DEPA, NETA, H$_2$macropa, Crown, PCTA, NODAGA, NODASA, Sar bicyclic chelator, MANOTA, and derivatives thereof.

(14) The complex according to (12), wherein the linear chelating agent is selected from a group consisting of HOPO, DTPA, MAG$_2$-GABA, Trisuccin, DFO, H$_2$dedpa, H$_4$octapa, HYNIC, HBED-CC, THP, and derivatives thereof.

(15) A pharmaceutical composition for treatment, prevention, and/or diagnosis of a cancer, comprising, as an active ingredient, the complex according to any one of (1) to (14).

(16) The pharmaceutical composition according to (15), wherein the cancer is a cancer expressing the CAPRIN-1 protein on a cell membrane surface.

(17) The pharmaceutical composition according to (15) or (16), wherein the cancer is breast cancer, renal cancer, pancreatic cancer, colorectal cancer, lung cancer, brain tumor, stomach cancer, uterine cancer, ovarian cancer, prostate cancer, bladder cancer, esophageal cancer, leukemia, lymphoma, liver cancer, gallbladder cancer, bile duct cancer, sarcoma, mastocytoma, melanoma, adrenocortical carcinoma, Ewing's tumor, Hodgkin's lymphoma, mesothelioma, multiple myeloma, testicular cancer, thyroid cancer, head and neck cancer, or urothelial cancer.

(18) A method of treating, preventing, and/or diagnosing a cancer, the method comprising administering the complex according to any one of (1) to (14) or the pharmaceutical composition according to any one of (15) to (17) to a subject.

Advantageous Effects of Invention

The complex according to the present invention not only exerts an extremely stronger anti-tumor effect than an antibody against a CAPRIN-1 protein or a fragment thereof used alone but also has an anti-tumor effect and/or a cancer detection capability that are much superior to those of existing radiopharmaceuticals. Thus, the complex according to the present invention can be utilized as an active ingredient in a pharmaceutical composition for treatment, prevention, and/or diagnosis of a cancer.

Description of Embodiments

The anti-tumor activity of the complex of an antibody against a CAPRIN-1 protein or a fragment thereof (hereinafter referred to as "anti-CAPRIN-1 antibody") and a radioisotope, the complex being used in the present invention, can be evaluated by examining the inhibition of tumor growth in a tumor-bearing animal *in vivo* as mentioned later. Moreover, detection of a cancer by the complex described above can be evaluated by administering the complex to a tumor-bearing animal *in vivo,* and measuring the dose of radiation from the complex in which the antibody against the CAPRIN-1 protein or the fragment thereof is labeled with the radioisotope, as well as the positional information of the radiation, as mentioned later.

In the present invention, "complex" refers to a complex in which a radioisotope and a chelating agent bound to an antibody are linked by a coordination bond. The antibody and the chelating agent may be bound via a linker.

The anti-CAPRIN-1 antibody forming the above-described complex according to the present invention may be a monoclonal antibody or a polyclonal antibody, and is preferably a monoclonal antibody. The complex of the present invention may be any type of antibody, as long as the complex can exhibit anti-tumor activity or a cancer detection capability. The antibody may be a recombinant antibody, a human antibody, a humanized antibody, a chimeric antibody, or a non-human animal antibody.

The radioisotope forming the above-described complex according to the present invention is an element with the capability of radiating radiation (radioactivity), the element being also called a radioactive isotope or a radioactive nuclide. The radioisotope used in the present invention is not particularly limited as long as the radioisotope is a radioisotope having the capability of radiating radiation useful for treatment, prevention, and/or diagnosis of a cancer.

A subject in need of detection of a cancer and treatment and/or prevention of a cancer in the present invention is a mammalian animal such as a human, a pet animal, a livestock animal, or a sport animal. Such a preferred subject is a human.

The anti-CAPRIN-1 antibody, the radioisotope, the complex of the anti-CAPRIN-1 antibody and the radioisotope, the pharmaceutical composition comprising the complex, the method of treating and/or preventing a cancer, and the method of detecting a cancer, related to the present invention, are described below.

<Anti-CAPRIN-1 Antibody>

Among CAPRIN-1 proteins having immunological reactivity with the anti-CAPRIN-1 antibody used in the present invention and having an amino acid sequence shown in any one of the even numbered SEQ ID NOs: 2 to 30, the amino acid sequences shown in SEQ ID NOs: 6, 8, 10, 12 and 14 are amino acid sequences of canine CAPRIN-1 proteins; the amino acid sequences shown in SEQ ID NOs: 2 and 4 are amino acid sequences of human CAPRIN-1 proteins; the amino acid sequence shown in SEQ ID NO: 16 is an amino acid sequence of a bovine CAPRIN-1 protein; the amino acid sequence shown in SEQ ID NO: 18 is an amino acid sequence of a horse CAPRIN-1 protein; the amino acid sequences shown in SEQ ID NOs: 20 to 28 are amino acid sequences of mouse CAPRIN-1 proteins; and the amino acid sequence shown in SEQ ID NO: 30 is an amino acid sequence of a chicken CAPRIN-1 protein.

The anti-CAPRIN-1 antibody used in the present invention may have immunological reactivity with a CAPRIN-1 protein variant having 80% or more, preferably 90% or more, more preferably 95% or more, and further preferably 99% or more sequence identity with the amino acid sequence shown in any one of the even numbered SEQ ID NOs: 2 to 30. The term "% sequence identity" as used herein means the percentage (%) of the number of identical amino acids (or nucleotides) to the total number of amino acids (or nucleotides) in a case in which two sequences are aligned such that maximum similarity can be achieved with or without introduction of gaps.

In the present invention, the anti-CAPRIN-1 antibody used for producing the complex means an antibody or a fragment thereof having immunological reactivity with a full-length CAPRIN-1 protein or a fragment thereof. The term "immunological reactivity" used herein means the binding characteristics between an antibody and a CAPRIN-1 protein or a partial polypeptide thereof *in vivo,* and is also referred to as "antigen-binding property".

The anti-CAPRIN-1 antibody used in the present invention may be a monoclonal antibody or a polyclonal antibody. The anti-CAPRIN-1 antibody may be any classes of immunoglobulin molecules, for example, IgG, IgE, IgM, IgA, IgD, or IgY, or any subclasses thereof, for example, IgG1, IgG2, IgG3, IgG4, IgA1, IgA2, or the like.

Polyclonal antibodies having immunological reactivity with a full-length CAPRIN-1 protein or a fragment thereof (anti-CAPRIN-1 polyclonal antibodies) can be obtained, for example, in a manner described below. Serum is collected from mice, human antibody-producing mice, rats, rabbits, chickens, or the like immunized with a naturally occurring CAPRIN-1 protein, the protein fused with GST or the like, or a partial peptide thereof. Then, the obtained serum can be subjected to, for example, ammonium sulfate precipitation, protein A, protein G, DEAE ion-exchange columns, affinity columns to which the CAPRIN-1 protein or the partial peptide is coupled.

As for the full-length CAPRIN-1 protein or the fragment thereof used in the immunization, the nucleotide sequences and amino acid sequences of CAPRIN-1 and homologs thereof can be obtained by, for example, accessing the website of GenBank (NCBI, USA) and using the BLAST or FASTA algorithm (Karlin and Altschul, Proc. Natl. Acad. Sci. USA, 90: 5873-5877, 1993; and Altschul et al., Nucleic Acids Res. 25: 3389-3402, 1997). Methods of producing the CAPRIN-1 protein can be obtained with reference to WO2014/012479 or may employ cells or the like expressing the CAPRIN-1 protein.

Monoclonal antibodies having immunological reactivity with a full-length CAPRIN-1 protein or a fragment thereof (anti-CAPRIN-1 monoclonal antibodies) can be obtained, for example, in a manner described below. A breast cancer cell SK-BR-3 expressing CAPRIN-1, a full-length CAPRIN-1 protein or a fragment thereof, or the like is administered to a mouse for immunization. Spleen cells separated from the mouse are fused with myeloma cells. Then, clones capable of producing anti-CAPRIN-1 monoclonal antibodies are selected from the resulting fusion cells (hybridomas) to obtain these antibodies. The antibodies produced from the selected hybridomas can be obtained in the same way as the aforementioned method of purifying polyclonal antibodies.

The antibody used in the present invention includes human antibodies, humanized antibodies, chimeric antibodies, and non-human animal antibodies.

For human antibodies, human lymphocytes infected with EB virus are sensitized with a protein, protein-expressing cells, or a lysate thereof. The sensitized lymphocytes are fused with human-derived myeloma cells such as U266 cells. Antibodies having immunological reactivity with a full-length CAPRIN-1 protein or a fragment thereof can be obtained from the resulting fusion cells.

A humanized antibody is an engineered antibody, which is also referred to as a reshaped human antibody. A humanized antibody is constructed by transplanting complementarity determining regions of an immunized animal-derived antibody into complementarity determining regions of a human antibody. In addition, a general gene recombinant technique thereof is well known. Specifically, a DNA sequence(s) designed in a manner that allows complementarity determining regions of mouse or rabbit antibody to be ligated to framework regions of human antibody is synthesized by the PCR method using several oligonucleotides prepared in such a manner that the oligonucleotides have portion(s) overlapping each other at end(s) of each thereof. A humanized antibody can be obtained by ligating the synthesized DNA to DNA encoding a human antibody constant region, incorporating the resultant into an expression vector, and introducing the vector into a host for antibody production (see EP239400 and WO96/02576). Framework regions of human antibody ligated via complementarity determining regions are selected such that complementarity determining regions can form a favorable antigen binding site. If necessary, amino acids in framework regions of an antibody variable region may be substituted in such a manner that complementarity determining regions in a reshaped human antibody form an appropriate antigen binding site (Sato K. et al., Cancer Research 1993, 53: 851-856). In addition, the framework regions may be substituted with framework regions derived from a different human antibody (see WO99/51743).

Specific examples of the fragment of the anti-CAPRIN-1 antibody include $F(ab')_2$, Fab', Fab, and scFv which are fragments having structures with antigen-binding properties in anti-CAPRIN-1 antibody fullbody. The fragments can be produced by a known method.

In general, antibodies are heteromultimeric glycoproteins comprising at least two heavy chains and two light chains. Antibodies are composed of two identical light chains and two identical heavy chains. Heavy chain has a heavy-chain variable region at one end thereof followed by some constant regions. Light chain has a light-chain variable region at one end thereof followed by some constant regions. Variable regions confer binding specificity to an antibody via a specific variable region, which is called complementarity determining region (CDR). A relatively conserved portion in a variable region is called a framework region (FR). A complete heavy-chain and light-chain variable region comprise 4 FRs connected via 3 CDRs (CDR1 to CDR3), respectively.

Sequences of constant regions and variable regions of human-derived heavy-chain and light-chain can be obtained from, for example, NCBI (USA; GenBank, UniGene, etc.). For example, for a human IgG1 heavy-chain constant region, see registration No. J00228; for a human IgG2 heavy-chain constant region, see registration No. J00230; for a human light chain κ constant region, see sequences such as registration Nos. V00557, X64135, and X64133; and for a human light chain λ constant region, see sequences such as registration Nos. X64132 and X64134.

A chimeric antibody is an antibody produced by combining sequences derived from different animals. An example thereof is an antibody consisting of variable regions of mouse antibody heavy-chain and light-chain and constant regions of human antibody heavy-chain and light-chain variable regions. Such a chimeric antibody can be produced by a known method. For example, such a chimeric antibody can be obtained by ligating DNA encoding an antibody V region to DNA encoding a human antibody C region, incorporating the resultant into an expression vector, and introducing the vector into a host for antibody production.

Non-human animal antibodies are obtained by immunizing animals with sensitizing antigens according to a known method. As a general method, sensitizing antigens are injected intraperitoneally, intracutaneously, or subcutaneously into animals such as mice. For injecting sensitizing antigens, an appropriate amount of various adjuvants including CFA (Freund's complete adjuvant) is mixed therewith and the mixture is administered to animals several times. After immunization of animals and confirmation of an anti-CAPRIN-1 antibody contained in serum, the serum is collected and the antibody can be obtained, for example, via ammonium sulfate precipitation, protein A, protein G, DEAE ion-exchange columns, affinity columns to which the CAPRIN-1 protein or the partial peptide is coupled, as mentioned above. In the case of obtaining monoclonal antibodies from non-human animals, a monoclonal antibody is obtained by collecting immunocytes from the immunized animals and fusing the immunocytes with myeloma cells. The cell fusion of immunocytes with myeloma cells can be carried out according to a known method (see Kohler, G. and Milstein, C. Methods Enzymol. (1981) 73, 3-46).

The antibody used in the present invention can also be obtained by cloning an antibody gene from a hybridoma, incorporating the gene into an adequate vector, introducing the vector into a host, and producing the antibody as a gene recombinant antibody by using a gene recombinant technique (see Carl, A.K. Borrebaeck, James, W. Larrick, THERAPEUTIC MONOCLONAL ANTIBODIES, Published in the United Kingdom by MACMILLAN PUBLISHERS LTD, 1990).

Amino acids in a variable region (e.g., FR) and/or a constant region in the anti-CAPRIN-1 antibody may be substituted with different amino acids to obtain the complex used in the present invention. The amino acid substitution is a substitution of 1 or several, for example, less than 15, less than 10, not more than 8, not more than 6, not more than 5, not more than 4, not more than 3, or not more than 2 amino acids, preferably 1 to 9 amino acids. A substituted antibody should have characteristics of specifically binding to the antigen and binding affinity for the antigen equivalent to or more than those of an unsubstituted antibody and should be an antibody that causes no rejection when applied to humans.

The anti-CAPRIN-1 antibody used in the present invention is expected to have a stronger anti-tumor effect when having higher binding affinity for the CAPRIN-1 protein on a cancer cell surface. It is desirable that an association constant (affinity constant) Ka (kon/koff) is preferably at least $10^7$ M$^{-1}$, at least $10^8$ M$^{-1}$, at least $5 \times 10^8$ M$^{-1}$, at least $10^9$ M$^{-1}$, at least $5 \times 10^9$ M$^{-1}$, at least $10^{10}$ M$^{-1}$, at least $5 \times 10^{10}$ M$^{-1}$, at least $10^{11}$ M$^{-1}$, at least $5 \times 10^{11}$ M$^{-1}$, at least $10^{12}$ M$^{-1}$, or at least $10^{13}$ M$^{-1}$.

The binding strength of the anti-CAPRIN-1 antibody used in the present invention against effector cells can be improved by substituting 1, 2, or several amino acids in the heavy chain-constant region of the antibody or by removing fucose bound to N-acetylglucosamine in an N-glycoside-linked sugar chain bound to the heavy-chain constant region. The anti-CAPRIN-1 antibody described above may have the amino acid substitution alone or may be a composition with an antibody bound to fucose.

Antibodies in which 1, 2, or several amino acids in the heavy-chain constant region have been substituted can be produced with reference to, for example, WO2004/063351, WO2011/120135, U.S. Patent No. 8388955, WO2011/005481, U.S. Patent No. 6737056, and WO2005/063351.

Antibodies in which fucose bound to N-acetylglucosamine in an N-glycoside-linked sugar chain in the heavy-chain constant region has been removed, or producing cells thereof, can be produced with reference to U.S. Patent No. 6602684, EP Patent No. 1914244, and U.S. Patent No. 7579170. Compositions of antibodies in which fucose bound to N-acetylglucosamine in an N-glycoside-linked sugar chain bound to the heavy-chain constant region has been removed, and antibodies bound to fucose, or producing cells thereof can be produced with reference to, for example, U.S. Patent No. 8642292.

The anti-CAPRIN-1 polyclonal antibody and the anti-CAPRIN-1 monoclonal antibody used in the present invention, methods for producing or purifying antibodies and methods for producing the CAPRIN-1 protein or partial polypeptide thereof used in immunization can be obtained with reference to WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176, and WO2015/020212.

Specific examples of the anti-CAPRIN-1 antibody according to the present invention include anti-CAPRIN-1 antibodies described in aforementioned WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212. Preferred examples of the anti-CAPRIN-1 antibody include the following.

An antibody or a fragment thereof having an immunological reactivity with a partial polypeptide of CAPRIN-1 protein having the amino acid sequence shown in SEQ ID NO: 2 or SEQ ID NO: 4 or an amino acid sequence having 80% or more (preferably 85% or more, more preferably 90% or more, further preferably 95% or more, and still further preferably 99% or more) sequence identity to the amino acid sequence.

An antibody or a fragment thereof having an immunological reactivity with a partial polypeptide of CAPRIN-1 protein having the amino acid sequence shown in SEQ ID NO: 31 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 36, 37, and 38 (CDR1, CDR2, and CDR3, respectively) and a light-chain variable region comprising complementarity determining regions of SEQ ID NOs: 40, 41, and 42 (CDR1, CDR2, and CDR3, respectively), and having an immunological reactivity with the CAPRIN-1 protein. Alternatively, an antibody or a fragment thereof comprising a heavy-chain variable region comprising complementarity determining regions of SEQ ID NOs: 140, 141, and 142 (CDR1, CDR2, and CDR3, respectively) and a light-chain variable region comprising complementarity determining regions of SEQ ID NOs: 143, 144, and 145 (CDR1, CDR2, and CDR3, respectively), and having an immunological reactivity with the CAPRIN-1 protein. Alternatively, an antibody or a fragment thereof comprising a heavy-chain variable region comprising complementarity determining regions of SEQ ID NOs: 164, 165, and 166 (CDR1, CDR2, and CDR3, respectively) and a light-chain variable region comprising complementarity determining regions of SEQ ID NOs: 167, 168, and 169 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 39 and a light-chain variable region comprising the amino acid sequence of SEQ ID NO: 43. Alternatively, an antibody or a fragment thereof comprising a heavy-chain variable region comprising the amino acid sequence of SEQ ID NO: 70 and a light-chain variable region comprising the amino acid sequence of SEQ ID NO: 71. Alternatively, an antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof having an immunological reactivity with a partial polypeptide of CAPRIN-1 protein having the amino acid sequence shown in SEQ ID NO: 33 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 60, 61, and 62 (CDR1, CDR2, and CDR3, respectively) and a light-chain variable region comprising complementarity determining regions of SEQ ID NOs: 64, 65, and 66 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 63 and a light-chain variable region comprising the amino acid sequence of SEQ ID NO: 67.

An antibody or a fragment thereof having an immunological reactivity with a partial polypeptide of CAPRIN-1 protein having the amino acid sequence shown in SEQ ID NO: 32 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 52, 53, and 54 (CDR1, CDR2, and CDR3, respectively) and a light-chain variable region comprising complementarity determining regions of SEQ ID NOs: 56, 57, and 58 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 55 and a light-chain variable region comprising the amino acid sequence of SEQ ID NO: 59.

An antibody or a fragment thereof having an immunological reactivity with a partial polypeptide of CAPRIN-1 protein having the amino acid sequence shown in SEQ ID NO: 34 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 170, 171, and 172 (CDR1, CDR2, and CDR3, respectively) and a light-chain variable region comprising complementarity determining regions of SEQ ID NOs: 173, 174, and 175 (CDR1, CDR2, and CDR3, respectively), and having an immunological reactivity with the CAPRIN-1 protein. Alternatively, an antibody or a fragment thereof comprising a heavy-chain variable region comprising complementarity determining regions of SEQ ID NOs: 176, 177, and 178 (CDR1, CDR2, and CDR3, respectively) and a light-chain variable region comprising complementarity determining regions of SEQ ID NOs: 179, 180, and 181 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 80 and a light-chain variable region comprising the amino acid sequence of SEQ ID NO: 81. Alternatively, an antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof having an immunological reactivity with a partial polypeptide of CAPRIN-1 protein having the amino acid sequence shown in SEQ ID NO: 35 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 182, 183, and 184 (CDR1, CDR2, and CDR3, respectively) and a light-chain variable region comprising complementarity determining regions of SEQ ID NOs: 185, 186, and 187 (CDR1, CDR2, and CDR3, respectively), and having an immunological reactivity with the CAPRIN-1 protein. Alternatively, an antibody or a fragment thereof comprising a heavy-chain variable region comprising complementarity determining regions of SEQ ID NOs: 188, 189, and 190 (CDR1, CDR2, and CDR3, respectively) and a light-chain variable region comprising complementarity determining regions of SEQ ID NOs: 191, 192, and 193 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 84 and a light-chain variable region comprising the amino acid sequence of SEQ ID NO: 85. Alternatively, an antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising complementarity determining regions of SEQ ID NOs: 44, 45, and 46 (CDR1, CDR2, and CDR3, respectively) and a light-chain variable region comprising complementarity determining regions of SEQ ID NOs: 48, 49, and 50 (CDR1, CDR2, and CDR3, respectively), and having an immunological reactivity with the CAPRIN-1 protein. Preferably, an antibody or a fragment thereof comprising a heavy-chain variable region comprising the amino acid sequence of SEQ ID NO: 47 and a light-chain variable region comprising the amino acid sequence of SEQ ID NO: 51.

An antibody or a fragment thereof having an immunological reactivity with a partial polypeptide of CAPRIN-1 protein having the amino acid sequence shown in SEQ ID NO: 296 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 146, 147, and 148 (CDR1, CDR2, and CDR3, respectively) and a light-chain variable region comprising complementarity determining regions of SEQ ID NOs: 149, 150, and 151 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof having an immunological reactivity with a partial polypeptide of CAPRIN-1 protein having the amino acid sequence shown in SEQ ID NO: 297 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 272, 273, and 274 (CDR1, CDR2, and CDR3, respectively) and a light-chain variable region comprising complementarity determining regions of SEQ ID NOs: 275, 276, and 277 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof having an immunological reactivity with a partial polypeptide of CAPRIN-1 protein having the amino acid sequence shown in SEQ ID NO: 298 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 290, 291, and 292 (CDR1, CDR2, and CDR3, respectively) and a light-chain variable region comprising complementarity determining regions of SEQ ID NOs: 293, 294, and 295 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof having an immunological reactivity with a partial polypeptide of CAPRIN-1 protein having the amino acid sequence shown in SEQ ID NO: 299 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 301, 302, and 303 (CDR1, CDR2, and CDR3, respectively) and a light-chain variable region comprising complementarity determining regions of SEQ ID NOs: 305, 306, and 307 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 300 and a light-chain variable region comprising the amino acid sequence of SEQ ID NO: 304.

An antibody or a fragment thereof having an immunological reactivity with a partial polypeptide of CAPRIN-1 protein having the amino acid sequence shown in SEQ ID NO: 308 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 134, 135, and 136 (CDR1, CDR2, and CDR3, respectively) and a light-chain variable region comprising complementarity determining regions of SEQ ID NOs: 137, 138, and 139 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof having an immunological reactivity with a partial polypeptide of CAPRIN-1 protein having the amino acid sequence shown in SEQ ID NO: 309 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 134, 135, and 136 (CDR1, CDR2, and CDR3, respectively) and a light-chain variable region comprising complementarity determining regions of SEQ ID NOs: 137, 138, and 139 (CDR1, CDR2, and CDR3, respectively), and having an immunological reactivity with the CAPRIN-1 protein. More preferably, an antibody or a fragment thereof comprising a heavy-chain variable region comprising the amino acid sequence of SEQ ID NO: 68 and a light-chain variable region comprising the amino acid sequence of SEQ ID NO: 69.

In addition, the following anti-CAPRIN-1 antibodies are preferably used.

An antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof comprising a heavy-chain variable region comprising the amino acid sequence of SEQ ID NO: 70 and a light-chain variable region comprising the amino acid sequence of SEQ ID NO: 71.

An antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof comprising a heavy-chain variable region comprising the amino acid sequence of SEQ ID NO: 74 and a light-chain variable region comprising the amino acid sequence of SEQ ID NO: 75.

An antibody or a fragment thereof comprising a heavy-chain variable region comprising the amino acid sequence of SEQ ID NO: 76 and a light-chain variable region comprising the amino acid sequence of SEQ ID NO: 77.

An antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof comprising a heavy-chain variable region comprising the amino acid sequence of SEQ ID NO: 80 and a light-chain variable region comprising the amino acid sequence of SEQ ID NO: 81.

An antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof comprising a heavy-chain variable region comprising the amino acid sequence of SEQ ID NO: 84 and a light-chain variable region comprising the amino acid sequence of SEQ ID NO: 85.

An antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising the amino acid sequence of SEQ ID NO: 88 and a light-chain variable region comprising the amino acid sequence of SEQ ID NO: 89.

An antibody or a fragment thereof comprising a heavy-chain variable region comprising the amino acid sequence of SEQ ID NO: 90 and a light-chain variable region comprising the amino acid sequence of SEQ ID NO: 91.

An antibody or a fragment thereof comprising a heavy-chain variable region comprising the amino acid sequence of SEQ ID NO: 92 and a light-chain variable region comprising the amino acid sequence of SEQ ID NO: 93.

An antibody or a fragment thereof comprising a heavy-chain variable region comprising the amino acid sequence of SEQ ID NO: 94 and a light-chain variable region comprising the amino acid sequence of SEQ ID NO: 95.

An antibody or a fragment thereof comprising a heavy-chain variable region comprising the amino acid sequence of SEQ ID NO: 96 and a light-chain variable region comprising the amino acid sequence of SEQ ID NO: 97.

An antibody or a fragment thereof comprising a heavy-chain variable region comprising the amino acid sequence of SEQ ID NO: 98 and a light-chain variable region comprising the amino acid sequence of SEQ ID NO: 99.

An antibody or a fragment thereof comprising a heavy-chain variable region comprising the amino acid sequence of SEQ ID NO: 100 and a light-chain variable region comprising the amino acid sequence of SEQ ID NO: 101.

An antibody or a fragment thereof comprising a heavy-chain variable region comprising the amino acid sequence of SEQ ID NO: 102 and a light-chain variable region comprising the amino acid sequence of SEQ ID NO: 103.

An antibody or a fragment thereof comprising a heavy-chain variable region comprising the amino acid sequence of SEQ ID NO: 104 and a light-chain variable region comprising the amino acid sequence of SEQ ID NO: 105.

An antibody or a fragment thereof comprising a heavy-chain variable region comprising the amino acid sequence of SEQ ID NO: 106 and a light-chain variable region comprising the amino acid sequence of SEQ ID NO: 107.

An antibody or a fragment thereof comprising a heavy-chain variable region comprising the amino acid sequence of SEQ ID NO: 108 and a light-chain variable region comprising the amino acid sequence of SEQ ID NO: 109.

An antibody or a fragment thereof comprising a heavy-chain variable region comprising the amino acid sequence of SEQ ID NO: 110 and a light-chain variable region comprising the amino acid sequence of SEQ ID NO: 111.

An antibody or a fragment thereof comprising a heavy-chain variable region comprising the amino acid sequence of SEQ ID NO: 112 and a light-chain variable region comprising the amino acid sequence of SEQ ID NO: 113.

An antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof comprising a heavy-chain variable region comprising the amino acid sequence of SEQ ID NO: 116 and a light-chain variable region comprising the amino acid sequence of SEQ ID NO: 117.

An antibody or a fragment thereof comprising a heavy-chain variable region comprising the amino acid sequence of SEQ ID NO: 118 and a light-chain variable region comprising the amino acid sequence of SEQ ID NO: 119.

An antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof comprising a heavy-chain variable region comprising the amino acid sequence of SEQ ID NO: 122 and a light-chain variable region comprising the amino acid sequence of SEQ ID NO: 123.

An antibody or a fragment thereof comprising a heavy-chain variable region comprising the amino acid sequence of SEQ ID NO: 124 and a light-chain variable region comprising the amino acid sequence of SEQ ID NO: 125.

An antibody or a fragment thereof comprising a heavy-chain variable region comprising the amino acid sequence of SEQ ID NO: 126 and a light-chain variable region comprising the amino acid sequence of SEQ ID NO: 127.

An antibody or a fragment thereof comprising a heavy-chain variable region comprising the amino acid sequence of SEQ ID NO: 128 and a light-chain variable region comprising the amino acid sequence of SEQ ID NO: 129.

An antibody or a fragment thereof comprising a heavy-chain variable region comprising the amino acid sequence of SEQ ID NO: 130 and a light-chain variable region comprising the amino acid sequence of SEQ ID NO: 131.

An antibody or a fragment thereof comprising a heavy-chain variable region comprising the amino acid sequence of SEQ ID NO: 132 and a light-chain variable region comprising the amino acid sequence of SEQ ID NO: 133.

An antibody or a fragment thereof comprising a heavy-chain variable region comprising the amino acid sequence of SEQ ID NO: 300 and a light-chain variable region comprising the amino acid sequence of SEQ ID NO: 304.

<Radioisotope>

The radioisotope used in the present invention is not particularly limited as long as the radioisotope has the capability of radiating radiation useful in treatment, prevention, and/or diagnosis of a cancer. A radioisotope that radiates an $\alpha$ (alpha) ray or a $\beta$ (beta) ($\beta$+ or $\beta$-) ray which is a particle radiation or a $\gamma$ (gamma) ray which is an electromagnetic radiation is preferably used as the radioisotope.

Specific examples of preferred radioisotopes include radium (Ra), thorium (Th), actinium (Ac), lutetium (Lu), indium (In), gallium (Ga), lead (Pb), copper (Cu), strontium (Sr), yttrium (Y), samarium (Sm), rhenium (Re), bismuth (Bi), technetium (Tc), and zirconium (Zr). Specific examples of more preferred radioisotopes include 227-Th, 225-Ac, 177-Lu, 111-In, 67-Ga, 68-Ga, 212-Pb, 64-Cu, 67-Cu, 89-Sr, 90-Y, 153-Sm, 186-Re, 188-Re, 212-Bi, 213-Bi, 99m-Tc, and 89-Zr.

Specific examples of the radioisotope that radiates an $\alpha$ ray include 223-Ra, 227-Th, 225-Ac, 212-Pb, 186-Re, 188-Re, 212-Bi, and 213-Bi. The energy of the $\alpha$ ray from the radioisotope that radiates the $\alpha$ ray is preferably 5 to 8 MeV.

Among radioisotopes radiating $\beta$ rays, specific examples of the radioisotope that radiates a $\beta$- ray include 177-Lu, 64-Cu, 67-Cu, 89-Sr, 90-Y, and 153-Sm. Specific examples of radioisotopes radiating $\beta$+ rays include 64-Cu, 68-Ga, and 89-Zr. The energy resulting from the radioisotope radiating a $\beta$+ ray is preferably 0.5 to 2.3 MeV.

Specific examples of the radioisotope that radiates a $\gamma$ ray include 111-In, 67-Ga, 177-Lu, and 99m-Tc. The energy resulting from the radioisotope radiating a $\gamma$ ray is preferably 0.1 to 0.5 MeV.

<Chelating Agent>

A chelating agent that forms a complex together with a radioisotope by a coordination bond and enables an anti-CAPRIN-1 antibody and the complex to be bound directly or via a linker is preferably used as the chelating agent used in the present invention. Specifically, the chelating agent is a bifunctional chelating agent that has both a protein-binding site and a site at which a complex with a radioisotope is formed. More preferably, the chelating agent is a chelating agent having a complex formation site that is cyclic or linear.

Specific examples of bifunctional cyclic chelating agents include, but are not limited to, DOTA, NOTA, TETA, TCMC, DEPA, NETA, $H_2$macropa, Crown, PCTA, NODAGA, NODASA, Sar bicyclic chelator, MANOTA, and derivatives thereof.

Specific examples of bifunctional linear chelating agents include, but are not limited to, HOPO, DTPA, MAG$_2$-GABA, Trisuccin, DFO, $H_2$dedpa, $H_4$octapa, HYNIC, HBED-CC, THP, and derivatives thereof.

A chelating agent that easily forms a complex together with a radioisotope is preferably used as the chelating agent. For example, $H_2$macropa is preferred in the case of using 223-Ra, $H_2$macropa, DOTA, or Crown is preferred in the case of using 225-Ac, DOTA or Me-3, or 2-HOPO is preferred in the case of using 227-Th, DOTA or TCMC is preferred in the case of using 212-Pb, 3p-C-DEPA, NETA, or CHX-A-DTPA is preferred in the case of using 212-Bi or 213-Bi, DOTA, DOTATATE, DOTANOC, NETA, NODAGA, PCTA, DTPA, CHX-A-DTPA, 1B4M-DTPA, or $H_4$octapa is preferred in the case of using 177-Lu, DOTA, DOTATATE, DOTANOC, NETA, CHX-A-DTPA, DTPA, or 1B4M-DTPA is preferred in the case of using 90-Y, DOTA or NOTA is preferred in the case of using 67-Cu, TETA, DOTA, NOTA, p-SCN-Bn-Oxo-DO3A, p-SCN-Bn-Oxo-PCTA, NODAGA, Sar Bicyclic chelators, or MANOTA is preferred in the case of using 64-Cu, MAG$_2$-GABA, Trisuccin, or NYNIC is preferred in the case of using 188-Re, DFO, NOTA, DOTA, p-SCN-Bn-Oxo-DO3A, DFO, $H_2$CHXdedpa, or H4CXHoctapa is preferred in the case of using 67-Ga, DOTA, DOTATATE, NOTA, NODASA, DTPA, $H_2$dedpa, or HBED-CCTHP is preferred in the case of using 68-Ga, DOTA, DOTATATE, DOTANOC, NOTA, NODAGA, NODASA, CHX-A-DTPA, $H_4$octapa, DTPA, 1B4M-DTPA, $H_2$CHXdedpa, $H_4$CXHoctapa, or EDDA/HYNIC-TOC is preferred in the case of using 111-In, HYNIC, EDDA/HYNIC-TOC, or HBEC-CC is preferred in the case of using 99m-Tc, and DFO, DFO* (optical isomer of DFO), or DFOcyclo is preferred in the case of using 89-Zr.

Examples of the complex of the radioisotope and the chelating agent are described in Theranostics 2021, Vol. 11, Issue 13, Molecules 2022, 27, 3062, US2022/363768, US2019/091354, US2017/319721, US2015/110817, US2010/129315, US2008/138899, US2023/072421, US2022/125960, US2022/118123, US2017/340759, US2019/298865, US2011/250137, US2011/123444, EP1590005A, EP2239274A, WO2008/046463A, WO2008/028530A, EP1861127A, EP1700608A, WO2022/162210A, WO2022/157094A, WO2021/160708A, WO2012/104225A, WO2012/069410A, WO2011/110490A, US6183721, US5776894, US5753627, US5686410, US5650134, US2022/401593, US2022/378954, EP0515313A, WO2022/043557A, WO2022/043558A, EP4034176A, WO2023/152671A, WO2022/219569A, WO2022/136317A, WO2022/043556A, US2023/321285, US2023/338587, US2024/050597, US2023/357381, US2024/197926, US2024/197715, US2024/252693, WO2024/116053A, WO2024/121722A, EP4279092A, US2024/108766, US2024/156999, US2024/245582, WO2023/222557A, and WO2023/159229A. Each of these complexes can also be utilized as a component in the complex of the present invention.

<Method of Producing Complex>

The complex of the present invention may be in a form in which the complex(es) of a radioisotope and a chelating agent is directly bound to an anti-CAPRIN-1 antibody, or may be in a form in which the complex(es) of a chelating agent is bound to an anti-CAPRIN-1 antibody via a linker. The details of a method of binding via the linker can be carried out by a known method (see, for example, Greg T. Hermanson Bioconjugate Techniques, Third Edition, WO2004/010957, and WO2014/012479).

Examples of reactive groups attached to the anti-CAPRIN-1 antibody, the chelating agent, and the linker, for forming the complex of the present invention, include the following.

Examples of such a reactive group attached to an amino acid sequence of the anti-CAPRIN-1 antibody or a glycoprotein modified with an amino acid include primary amine ($\varepsilon$-amino), carboxyl, thiol (sulfhydryl), carbonyl (ketone or aldehyde), and hydroxyl, so long as these are not subjected to special chemical modification. The primary amine exists in the N-terminal of a polypeptide or the side chain of a lysine residue, has positive charge under physiological conditions, and commonly exists in the outside of a protein. Therefore, the primary amine can be used for binding without denaturing the structure of a protein. The carboxyl exists in the C-terminus of a polypeptide, or the side chain of aspartic acid or glutamic acid. The sulfhydryl exists in the side chain of cysteine, and forms a disulfide bond that maintains the higher-order structure of a protein. The ketone or aldehyde is produced in a glycoprotein by oxidizing glycosyl with sodium metaperiodate.

Specific examples of the reactive group attached to the chelating agent and the linker include the following.

N-hydroxysuccinimide (NHS) ester, imidoester, pentafluorophenyl ester, hydroxymethyl phosphine, isothiocyanate, isocyanate, acyl azide, N-hydroxyl ester, sulfonyl chloride, aldehyde, glyoxal, epoxide, oxirane, carbonate, aryl, carbodiimide, and carboxylic acid anhydride which are reactive groups that can react with amine.

Carbodiimide, diazoalkane, diazoacetyl compounds, carbonyldiimidazole which are reactive groups that can react with carboxyl and amine.

Maleimide, haloacetamide, pyridyl disulfide, thiosulfone, vinyl sulfone, haloacetyl, aziridine, acryloyl, and aryl, which are reactive groups that can react with thiol.

Hydrazide and alkoxyamine, which are reactive groups that can react with aldehyde. Epoxy, oxirane, carbonyldiimidazole, N,N'-disuccinimidyl carbonate, N-hydroxysuccinimidyl chloroformate, and isocyanate, which are reactive groups that can react with hydroxyl.

Isocyanate, which is a reactive group that can react with hydroxyl.

Diazirine, arylazide, aryl, benzophenol, and diazo compounds, which are reactive groups with photoreactivity.

Specific examples of linkers having the reactive groups described above include the following.

The linkers used as linkers having identical terminal reactive groups include a linker comprising N-hydroxysuccinimide ester as a reactive group (for example, Disuccinimidyl Glutarate (DSG), Disuccinimidyl Suberate (DSS), Bis(sulfosuccinimidyl)Suberate (BS3), Tris-(Succinimidyl)Aminotriacetate (TSAT), PEGylated Bis(Sulfosuccinimidyl) Suberate (BS(PEG)$_5$, BS(PEG)$_9$), Dithiobis(Succinimidyl Propionate) (DSP), 3,3'-dithiobis(sulfosuccinimidyl propionate) (DTSSP), ethylene glycol bis(succinimidyl succinate) (EGS), Sulfo-ethylene glycol bis(succinimidyl succinate (Sulfo-EGS), Dimethyl adipimidate 2HCl (DMA), Dimethyl pimelimidate 2HCl (DMP), Dimethyl suberimidate 2HCl (DMS), Dimethyl 3,3'-dithiobispropionimidate 2HCl (DTBP), 1,5-difluoro-2, 4-dinitrobenzene (DFDNB), Disuccinimidyl tartrate (DST), or Bis[2-(Succinimidooxycarbonyloxy)ethyl]Sulfone (BSOCOES), and a linker comprising maleimide as a reactive group (for example, Bismaleimidoethane (BMOE), 1,4-bismaleimidobutane (BMB), Bismaleimidohexane (BMH), Tris(2-maleimidothyl)amine (TMEA), 1,8-bismaleimido-(PEG)$_2$ (BM(PEG)$_2$), 1,8-bismaleimido-(PEG)$_3$ (BM(PEG)$_3$), or Dithiobismaleimidoethane (DTME).

The linkers used as major linkers having different terminal reactive groups include a linker comprising NHS ester and maleimide as reactive groups (for example, AMAS, BMPS, GMBS, Sulfo-MBS, MBS, Sulfo-MBS, SMCC, Sulfo-SMCC, EMCS, Sulfo-EMCS, SMPB, Sulfo-SMPB, SMPH, LC-SMCC, Sulfo-KMUS, SM(PEG)$_2$, SM(PEG)$_4$, SM(PEG)$_6$, SM(PEG)$_8$, SM(PEG)$_{12}$, or SM(PEG)$_{24}$), a linker comprising NHS ester and pyridyldithiol as reactive groups (for example, SPDP, LC-SPDP, Sulfo-LC-SPDP, SMPT, $(PEG)_4$SPDP, or PEG12-SPDP), a linker comprising NHS ester and haloacetyl as reactive groups (for example, SIA, SBAP, STAB, or Sulfo-SIAB), a linker comprising NHS ester and arylazide as reactive groups (for example, ANB-NOS, Sulfo-SANPAH, or ATFB), a linker comprising NHS ester and diazirine as reactive groups (for example, SDA, Sulfo-SDA, LC-SDA, SDAD, or Sulfo-SDAD), a linker comprising carbodiimide as a reactive group (for example, DCC, EDC, EDAC, NHS, or Sulfo-NHS), a linker comprising maleimide and hydrazide as reactive groups (for example, BMPH, EMCH, MPBH, or KMUH), a linker comprising pyridyldithiol and hydrazide as reactive groups (for example, PDPH), a linker comprising isocyanate and maleimide as reactive groups (for example, PMPI), and a linker comprising NHS ester and psoralen as reactive groups (for example, SPB).

The linker used as another linker includes a linker comprising a polypeptide, for example, Fmoc-Ala-Ala-Asn-PAB, Fmoc-Ala-Ala-Asn(Trt)-PAB, Fmoc-$PEG_3$-Ala-Ala-Asn(Trt)-PAB, Fmoc-$PEG_4$-Ala-Ala-Asn(Trt)-PAB, Fmoc-Ala-Ala-Asn-PAB-PNP, Fmoc-Ala-Ala-Asn(Trt)-PAB-PNP, Fmoc-$PEG_3$-Ala-Ala-Asn(Trt)-PAB-PNP, Azide-$PEG_4$-Ala-Ala-Asn(Trt)-PAB-PNP, Mal-$PEG_4$-Ala-Ala-Asn(Trt)-PAB-PNP, Fmoc-Val-Cit-PAB-OH, Val-Cit-PAB-OH, Fmoc-Val-Cit-PAB-PNP, MC-Val-Cit-PAB, MC-Val-Cit-PAB-PNP, or the like.

Additionally, Bis-PEG-acid, PEG Acid (for example, Acid-PEG-TEMPO, Amino-PEG-acid, Amino-PEG-$CH_2CO_2H$, Aminoxy-PEG-acid, Azido-PEG-acid, Carboxy-PEG-sulfonic acid, Fmoc-N-amido-PEG-acid, Fmoc-N-amido-PEG-$CH_2CO_2H$, Fmoc-aminooxy-PEG-acid, Hydroxy-PEG-acid, Hydroxy-PEG-$CH_2CO_2H$, m-PEG-acid, m-PEG-$(CH_2)_3$-acid, Methoxytrityl-N-PEG-acid, N-methyl-N-(t-Boc)-PEG-acid, Propargyl-PEG-acid, Propargyl-PEG-$CH_2CO_2H$, Propargyl-PEG-$(CH_2)_3$-acid, t-Boc-N-amido-PEG-acid, t-Boc-N-amido-PEG-$CH_2CO_2H$, t-Boc-Aminooxy-PEG-acid, Acid-PEG-PFP ester, Miscellaneous PEG acid), PEG PFP ester (for example, Acid-PEG-PFP ester, Bis-PEG-PFP ester), Bis-PEG-NHS, PEG Aldehyde (for example, m-PEG-aldehyde, m-PEG-benzaldehyde, Ald-PEG-acid, Ald-PEG-amine, Ald-PEG-azide, Ald-PEG-NH-Boc, Ald-PEG-NHS ester, Ald-PEG-TFP ester, Ald-PEG-t-butyl ester), PEG Tosylate (for example, Azido-PEG-Tos, Hydroxy-PEG-Tos, m-PEG-Tos, t-boc-Aminooxy-PEG-Tos, Trifluoroethyl-PEG-Tos, Tos-PEG-acid, Tos-PEG-$CH_2CO_2H$, Tos-PEG-alkyne, Tos-PEG-t-butyl ester, Tos-PEG-$CH_2CO_2tBu$, Tos-PEG-Tos, S-acetyl-PEG6-Tos, N-Tos-N-(t-butoxycarbonyl)-aminooxy-$PEG_4$-Tos, Ms-PEG-Ms, Ms-PEG-t-butyl ester, PEG-Ms, Propargyl-PEG-Ms), Boc-PEG (for example, Amino-PEG-t-Boc-Hydrazide, Azido-PEG-t-Boc-Hydrazide, Boc-NH-PEG-NH-Boc, Bromoacetamido-PEG-Boc-amine, m-PEG-ONHBoc, Mal-Alkyl-t-Boc-amine, N-Boc-PEG-alcohol, N-Boc-PEG-bromide, N-methyl-N-(t-Boc)-PEG-acid, t-Boc-N-amido-PEG-acid, t-Boc-N-amido-PEG-$CH_2CO_2H$, t-Boc-N-Amido-PEG-amine, t-Boc-N-amido-PEG-azide, t-Boc-N-amido-PEG-NHS ester, t-Boc-N-amido-PEG-sulfonic acid), PEG NHS ester (for example, Acid-PEG-NHS ester, Azido-PEG-NHS ester, Bis-PEG-NHS, Fmoc-PEG-NHS ester, m-PEG-NHS ester, m-PEG-NHS Carbonate, Mal-PEG-NHS ester, Propargyl-PEG-NHS ester, t-Boc-N-amido-PEG-NHS ester, t-Butoxycarbonyl-PEG-NHS ester), Fmoc-PEG (for example, Fmoc-N-amido-PEG-acid, Fmoc-NH-PEG-$CH_2CO_2H$, Fmoc-PEG-NHS ester), Biotin PEG (for example, Biotin PEG-acid, Biotin PEG-alcohol, Biotin PEG-alkyne, Biotin PEG-amine, Biotin PEG-azide, Biotin PEG-DBCO, Biotin PEG-hydrazide, Biotin-PEG-Mal, Biotin-PEG-NHS, Biotin-EDA-PEG-NHS, Biotin-PEG-oxyamine, Biotin-PEG-PFP, Biotin-EDA-PEG-PFP, Biotin-PEG-Tetrazine, Biotin-PEG-TFP, Azide-SS-biotin, Biotin-$PEG_3$-SS-azide, DBCO-S-S-$PEG_3$-Biotin, Dde Biotin-PEG4-Alkyne, Dde Biotin-$PEG_4$-Azide, Dde Biotin-$PEG_4$-DBCO, Diazo Biotin-$PEG_3$-Alkyne, Diazo Biotin-$PEG_3$-Azide, Diazo Biotin-$PEG_3$-DBCO, Diol Biotin-$PEG_3$-Alkyne, Diol Biotin-$PEG_3$-Azide, PC Biotin-$PEG_3$-Alkyne, PC-Biotin-$PEG_4$-$PEG_4$-Alkyne, PC-Biotin-$PEG_4$-PEG4-Alkyne, PC Biotin-$PEG_3$-Azide, PC-Biotin-PEG4-PEG3-Azide, PC-Biotin-$PEG_4$-NHS carbonate, PC DBCO-$PEG_3$-Biotin, WSPC Biotin-$PEG_3$-DBCO, Fmoc-Lys (biotin-PEG)-OH, Fmoc-N-amido-(PEG-biotin)-acid, TAMRA-Azide-PEG-Biotin), PEG Phosphonate, Aminooxy PEG (for example, Aminooxy-PEG-acid, Aminooxy-PEG-alcohol, Aminooxy-PEG-azide, Aminooxy-PEG-bromide, Aminooxy-PEG-methane, Aminooxy-PEG-Propargyl, Aminooxy-PEG-t-butyl ester, Aminooxy-PEG-Thiol, Bis-(Aminooxy)-PEG, t-Boc-Aminooxy-PEG-acid, t-Boc-Aminooxy-PEG-alcohol, t-Boc-Aminooxy-PEG-amine, t-Boc-Aminooxy-PEG-Azide, t-Boc-Aminooxy-PEG-Bromide, t-Boc-aminooxy-PEG-Methane, t-Boc-aminooxy-PEG-Propargyl, t-Boc-aminooxy-PEG-S-Ac, t-Boc-Aminooxy-PEG-Thiol, t-Boc-Aminooxy-PEG-Tos, Fmoc-aminooxy-PEG-acid, Trifluoroethyl-PEG-Aminooxy), Alkyne PEG (for example, endo-BCN-PEG, exo-BCN-PEG, Propargyl-PEG-acid, Propargyl-PEG-$CH_2CO_2H$, Propargyl-PEG-$(CH_2)_3$-acid, Propargyl-PEG-$(CH_2)_3$-methyl ester, Propargyl-PEG-Acrylate, Propargyl-PEG-alcohol, Propargyl-PEG-amine, Propargyl-PEG-methylamine, Aminooxy-PEG-Propargyl, Propargyl-PEG-azide, Propargyl-PEG-bromide, Propargyl-PEG-Maleimide, Propargyl-PEG-Ms, Propargyl-PEG-NHS ester, Propargyl-PEG-sulfonic acid, Propargyl-PEG-t-butyl ester, Propargyl-PEG-$CH_2CO_2tBu$, Propargyl-PEG-thiol, Propargyl-PEG-5-nitrophenyl carbonate, t-Boc-aminooxy-PEG-Propargyl, Bis-Propargyl-PEG, m-PEG-Propargyl), Azido PEG (for example, Azido-PEG-acid, Azido-PEG-$CH_2CO_2H$, Azido-PEG-$(CH_2)_3$-methyl ester, Azido-PEG-Acrylate, Azido-PEG-alcohol, Azido-PEG-$(CH_2)_3OH$, Azido-PEG-amine, Azido-PEG-azide, Azido-PEG-Maleimide, Azido-PEG-methylamine, Azido-PEG-methyl ester, Azido-PEG-NHS ester, Azido-PEG-$CH_2CO_2$-NHS, Azido-PEG-oxazolidin-2-one, Azido-PEG-PFP ester, Azido-PEG-phosphonic acid, Azido-PEG-phosphonic acid ethyl ester, Azido-PEG-sulfonic acid, Azido-PEG-t-Boc-Hydrazide, Azido-PEG-t-butyl ester, Azido-PEG-$CH_2CO_2$-t-butyl ester, Azido-PEG-TFP ester, Azido-PEG-Tos, Aminooxy-PEG-azide, Bromo-PEG-azide, Bromoacetamido-PEG-azide, Carboxyrhodamine 110-PEG-Azide, Isothiocyanato-PEG-Azide, Isothiocyanato-PEG-Azide, m-PEG-azide, Propargyl-PEG-azide, TAMRA-PEG-Azide, t-Boc-N-Amido-PEG-Azide, t-Boc-Aminooxy-PEG-Azide, Thiol-PEG-Azide, Trifluoroethyl-PEG-Azide, Azido-PEG-amino acid, Azido-$PEG_4$-4-nitrophenyl carbonate, S-Acetyl-$PEG_3$-Azido, Azide, Trityl-$PEG_{10}$-Azide), Alkyne PEG, DBCO-PEG, BCN-PEG, Propargyl-PEG, Bis-PEG-acid, Bis-PEG-NHS, Bis-PEG-PFP, Bis-Propargyl-PEG, Amine-PEG-Amine, Azido-PEG-azide, Bromo-PEG, or Mal PEG is used.

In another embodiment, a complex expected to allow kinetics *in vivo* to be more favorable can be obtained by using, as a linker, polyethylene glycol (PEG) described in WO2015/057699 or WO2017/165851.

The linker used in the present invention may be cleavable under intracellular conditions. A substance with anti-tumor activity, the substance comprising a radioisotope and a chelating agent, or a radioisotope, a chelating agent, and part of the linker, may be liberated in a cell. For example, the linker is a linker cleaved by peptidase or protease in a cell. The linker is preferably a linker cleaved by lysosome, endosomal protease, cathepsin B, cathepsin D, or plasmin. Examples of the linker include a linker comprising a polypeptide that can be cleaved by cathepsin B (Val-Cit, Phe-Leu, or Gly-Phe-Leu-Gly). In more detail, a linker described in U.S. Pat. No. 6,214,345 can be used.

The linker existing between the anti-CAPRIN-1 antibody and the radioisotope may be composed of one or more kinds of linkers.

In a method of producing the complex of the present invention, a functional group of the chelating agent is preferably bound to an ε-amino group or a thiol group which is originally possessed by an anti-CAPRIN-1 antibody or formed by chemical treatment.

In the case of using the ε-amino group, the ε-amino group of a lysine residue of the anti-CAPRIN-1 antibody can be used. In such a case, many lysine residues exist in the anti-CAPRIN-1 antibody, and therefore, binding to the chelating agent occurs nonspecifically.

In the case of using the thiol group, the thiol group can be produced from a disulfide bond in a cysteine residue of an anti-CAPRIN-1 antibody by using a reducing agent such as mercaptoethanol or dithiothreitol (DTT). In a method of forming thiol in an antibody, thiol may be formed by adding a thiol group to a primary amine in a lysine residue of an antibody by introducing Traut's reagent (2-iminothiolane or N-succinimidyl S-acetylthioacetate (SATA)). Alternatively, thiol may be formed by using THIOMAB (registered trademark) technology (see Nature Biotechnology 26, 925-932 (2008)) which is a method in which a predetermined number of thiol groups can be introduced into a specific moiety of an antibody, a method using ThioBridge (registered trademark) technology (see Methods Mol Biol. 2078: 113-129 (2020)), or RESPECT (registered trademark) technology (see MAbs. 9(6) 907-915 (2017)).

A thiol group added by cleaving the disulfide bond of an antibody by reduction treatment is preferably subjected to treatment (capping) for preventing formation of a disulfide bond again. For example, N-ethylmaleimide (NEM) or iodoacetamide (2-iodoacetamide (IAA)) can be used in the capping.

The formation of the complex by using a thiol group added to an antibody can be performed by a known method. Specifically, the binding can be performed by using, for example, a linker reagent having a maleimide group or a linker reagent having a bromoacetamide group as a linker reagent that specifically binds to a thiol group of an antibody subjected to reduction treatment. For example, N-succinimidyl-4-(N-maleimidomethyl)-cyclohexane-1-carboxylate (SMCC) is used as the linker having a maleimide group. In such a case, through an amino group existing in the chelating agent, the complex can be obtained by forming an amide bond with the N-succinimide group of SMCC.

In another embodiment, the complex can be obtained by forming an amide bond in an amino group existing in a chelating agent in advance by using SMCC, and then allowing reaction between thiol group added to an antibody side and a maleimide group of SMCC, to which a chelating agent is bound.

In another embodiment, an antibody and a chelating agent can also be bound by using two linkers. For example, a thiol group is added to the antibody by producing an amide bond between a primary amino group existing in a lysine residue in an antibody side and an N-succinimide group of SATA (N-succinimidyl-S-acetylthioacetate). Then, SMCC is allowed to react with a chelating agent having an amino group or a chelating agent added amino group according to a usual method resulting in formation of amide bond with an N-succinimide group in SMCC. The complex can be obtained by reaction between a maleimide group in SMCC to which the chelating agent is bound and the thiol group in SATA to which the antibody is bound.

In another embodiment, examples of a method of binding an antibody and a chelating agent include a method in which maleimidocaproyl-valine-citrulline-p-aminobenzyloxycarbonyl (me-Val-Cit-PAB) is used as a linker. mc-val-Cit-PAB (mc-vc-PAB) is a linker that can be cleaved by a protease (for example, cathepsin B) in a cell. By using DTT or the like, a thiol group is added to an antibody dissolved in a phosphate buffer. In parallel, benzyloxycarbonyl (PAB) in mc-Val-Cit-PAB is allowed to react with a chelating agent having an amino group, to produce the chelating agent to which mc-val-Cit-PAB is bound. The resultant is allowed to react with the antibody to which the thiol is added to obtain the complex.

In further another embodiment, SATA is bound to a primary amino group in a lysine residue of an antibody to add a thiol group. In parallel, succinimidyl 3-(2-pyridyldithio)propionate (SPDP) is allowed to react with a chelating agent having an amino group. Then, an amide bond is formed with an N-succinimide group in SPDP.

Example method for stabilizing binding via thiol includes a method using sulfone phenyl oxadiazole, a 4-cyanoethynyloxy derivative, or the like. The binding is stable for a longer period than binding by conjugate addition reaction of cysteine with maleimide. In a case in which an imide ring in which a thiol group is added to maleimide is opened by hydrolysis, to form an amide bond, stability is improved. Therefore, a linker having an amino group in the vicinity of an imido group can also be used. Since thiol of cysteine in an antibody forms a disulfide bond, a method in which a radioisotope is bound therebetween via two thiols is also conceivable. For example, a cross-linked bond can be formed by using dibromomaleimide or a linker having two disulfide binding sites that can be generated from an amide group having two sulfones at β-positions.

The amount of thiol added to an antibody can be quantified by, for example, mixing 5,5'-dithiobis(2-nitrobenzonic acid) (DTNB) and a sample solution comprising SH groups with a phosphate buffer (pH 8.0) and distilled water, adding a DTNB solution dissolved in a phosphate buffer, a Good's buffer, or a Tris buffer, incubating the resultant for a certain period, and then measuring an absorbance at 412 nm (see G. L. Ellman, Arch. Biochem. Biophys., 82, 70(1959)).

In another embodiment, for example, a linker having glucuronic acid (preferably, β-D-glucuronide) described in WO2007/0711968 can be used as means for improving the stability, solubility, and metabolic property of the complex of the present invention in blood, and the binding property of a chelating agent to an anti-CAPRIN-1 antibody. Alternatively, means described in WO2013/173337, WO2015/095755, WO2015/123679, or WO2018/031690 can be used.

In another embodiment, site-specific binding of a chelating agent to an anti-CAPRIN-1 antibody can be achieved by using, for example, means described in WO2006/65533 or WO2018/160683.

In a method of producing the complex of the present invention, it is acceptable to bind an anti-CAPRIN-1 antibody and a chelating agent and then form a complex of a radioisotope, or it is acceptable to form a complex of a radioisotope and a chelating agent and then bind the complex and an anti-CAPRIN-1 antibody. It is preferable to bind an anti-CAPRIN-1 antibody and a chelating agent and then form a complex of a radioisotope.

In the method to bind an anti-CAPRIN-1 antibody and a chelating agent and then forming a complex of a radioisotope, first, a reactant obtained by binding the anti-CAPRIN-1 antibody and the chelating agent by the above-mentioned method is subjected to, for example, gel filtration chromatography or the like. By fractionating a substance having the molecular weight larger than that of the antibody before the binding of the chelating agent, a complex in which the anti-CAPRIN-1 antibody and the chelating agent are bound is obtained. The complex in which the chelating agent and the anti-CAPRIN-1 antibody are bound, a radioisotope, and a buffer are mixed and allowed to react with each other to obtain the complex of the present invention.

Examples of a buffer that can be used as the buffer include, but are not limited to, a sodium acetate buffer, an ammonium acetate buffer, a HEPES buffer, a MES buffer, an ascorbic acid buffer, a gentisic acid buffer, and liquid mixtures thereof. The pH of the buffer is, for example, 1 to 11, preferably 3 to 9, and more preferably 4 to 7. A reaction temperature is, for example, 5 to 95°C, preferably 20 to 80°C, and more preferably 35 to 45°C. Examples of reaction time for which the reaction can be performed include, but are not limited to, 5 minutes to 48 hours, and more preferably 30 minutes to 24 hours.

<Physical Properties of the Complex>

The number of the molecules of the chelating agent bound per molecule of the anti-CAPRIN-1 antibody in the complex of the present invention can be quantified by a method such as mass spectrometry. In the case of using the complex of the present invention as an active ingredient of a pharmaceutical composition for treatment, prevention, and/or diagnosis of a cancer, the number of the molecules of the chelating agent per molecule of the anti-CAPRIN-1 antibody is preferably 0.5 to 20, and more preferably 1 to 10.

The radiochemical purity (%) of the complex of the present invention can be determined by a known method such as a thin-layer chromatography method, a radio TLC method, or a radio HPLC method. In the case of using the thin-layer chromatography method, specifically, 1) a reacted complex is spotted on the origin point of a thin-layer plate, 2) the complex is developed to the solvent front position of the thin-layer plate by using a developing solvent, 3) after the development, each of the lower and upper parts of the thin-layer plate obtained by cutting the thin-layer plate at a cutting position is put in a vial for measurement, and the amount of radiation is measured in a radiation measurement device such as an ionization chamber, and 4) the radiochemical purity is measured based on the following equation:

Radiochemical purity (%) = ((amount of radiation of lower part of thin-layer plate - background)/((amount of radiation of lower part of thin-layer plate - background) + (amount of radiation of upper part of thin-layer plate - background)) × 100)).

In the case of using the complex of the present invention as an active ingredient of a pharmaceutical composition for treatment, prevention, and/or diagnosis of a cancer, the radiochemical purity is 50% or more, preferably 60% or more, more preferably 70% or more, further preferably 80% or more, and most preferably 90% or more.

<Pharmaceutical Composition for Treatment, Prevention, and/or Diagnosis of Cancer>

The complex of the present invention can be used as an active ingredient of a pharmaceutical composition, particularly a pharmaceutical composition for treatment, prevention, and/or diagnosis of a cancer.

The terms "tumor" and "cancer" used herein refer to malignant neoplasm, and are thus used interchangeably.

A cancer that can be a target in the present invention is any cancer as long as the cancer expresses the CAPRIN-1 protein on a cell membrane surface. The cancer is preferably breast cancer, renal cancer, pancreatic cancer, colorectal cancer, lung cancer, brain tumor, stomach cancer, uterine cancer, ovarian cancer, prostate cancer, bladder cancer, esophageal cancer, leukemia, lymphoma, liver cancer, gallbladder cancer, sarcoma, mastocytoma, melanoma, adrenocortical carcinoma, Ewing's tumor, Hodgkin's lymphoma, mesothelioma, multiple myeloma, testicular cancer, thyroid cancer, head and neck cancer, or urothelial cancer, as described above.

More specific examples of the cancer include, but are not limited to, breast adenocarcinoma, complex-type breast adenocarcinoma, malignant mixed tumor of mammary gland, intraductal papillary adenocarcinoma, recurrent metastatic breast cancer, lung adenocarcinoma, non-small cell lung cancer (NSCLC), squamous non-small cell lung cancer, squamous cell cancer, small cell cancer, large cell cancer, glioma which is a neuroepithelial tissue tumor, glioblastoma, neuroblastoma, ependymoma, neuronal cellular tumor, embryonal neuroectodermal tumor, neurilemoma, neurofibroma, meningioma, chronic lymphocytic leukemia, lymphoma, alimentary lymphoma, gastrointestinal lymphoma, small to medium cell-type lymphoma, cecal carcinoma, ascending colon cancer, descending colon cancer, transverse colon cancer, sigmoid colon cancer, rectal cancer, ovarian epithelial cancer, germ cell tumor, stromal cell tumor, pancreatic ductal carcinoma, invasive pancreatic ductal carcinoma, adenocarcinoma of pancreatic cancer, metastatic adenocarcinoma, acinar cell carcinoma, adenosquamous carcinoma, giant cell tumor, intraductal papillary mucinous tumor, mucinous cystadenocarcinoma, pancreatoblastoma, pancreatic islet cell tumor, Frants tumor, serous cystadenocarcinoma, solid papillary carcinoma, gastrinoma, glucagonoma, insulinoma, multiple endocrine neoplasia type-1 (Wermer syndrome), nonfunctional islet cell tumor, somatostatinoma, VIPoma, cervical cancer, endometrial cancer, fibrosarcoma, osteogenic sarcoma, joint sarcoma, Ewing's sarcoma, Wilms' tumor, hepatoblastoma, soft tissue sarcoma, acute leukemia, chronic leukemia, spinal cord tumor, malignant soft tissue tumor, tumors of teratoma group, and head and neck cancer including hypopharyngeal cancer, oropharyngeal cancer, tongue cancer, epipharyngeal cancer, oral cancer, lip cancer, sinus cancer, laryngeal cancer, and recurrent brain tumor.

A preferable subject (patient) that can be a target is a mammalian animal, for example, a mammalian animal including primates, pet animals, livestock animals, and sport animals, and particularly preferably a human, a dog, or a cat.

The complex of the present invention has anti-tumor activity *in vitro* and/or *in vivo*. The anti-tumor activity means the decrease, elimination, inhibition of growth, apoptosis, necrosis, or killing of a cancer cell to be targeted. Thus, the effect of treatment and/or prevention of a cancer by the pharmaceutical composition comprising the complex of the present invention as an active ingredient can be evaluated by examining the anti-tumor activity against a cancer.

In order to determine an anti-tumor effect *in vivo*, the anti-tumor effect can be evaluated by administering the complex to a living organism having a cancer, measuring the size of a tumor after the administration, and examining the size of the cancer over time. Also, the anti-tumor effect can be evaluated by examining a survival rate. The anti-tumor effect can be further determined by examining prevention of a cancer, specifically, prevention of metastasis of a cancer, or prevention of recurrence of a cancer.

The complex of the present invention can be expected to have a stronger anti-tumor effect or cancer detection effect when having higher binding affinity for the CAPRIN-1 protein on a cancer cell surface. It is desirable that an association constant (affinity constant) Ka (kon/koff) is preferably at least $10^7$ M$^{-1}$, at least $10^8$ M$^{-1}$, at least $5 \times 10^8$ M$^{-1}$, at least $10^9$ M$^{-1}$, at least $5 \times 10^9$ M$^{-1}$, at least $10^{10}$ M$^{-1}$, at least $5 \times 10^{10}$ M$^{-1}$, at least $10^{11}$ M$^{-1}$, at least $5 \times 10^{11}$ M$^{-1}$, at least $10^{12}$ M$^{-1}$, or at least $10^{13}$ M$^{-1}$.

The capability of the complex of the present invention to bind to CAPRIN-1 can be specified by utilizing binding assay using, for example, surface plasmon resonance method (SPR method), ELISA, Western blot method, immunofluorescence, flow cytometry method, or the like.

The complex of the present invention exerts a stronger anti-tumor effect than an anti-tumor effect exerted by an anti-CAPRIN-1 antibody alone, as described above. By utilizing the functions of an anti-CAPRIN-1 antibody and a radioisotope, the complex can also be utilized as an active ingredient of a pharmaceutical composition for diagnosing whether a subject (patient) is suffering from the cancer described above. Specifically, the complex can detect not only the cancer tissue or cancer cell of a subject (patient) but also the position and size of the cancer tissue or cancer cell in the subject (patient) by administering the complex of the present invention into the body of the subject (patient) and detecting radiation from the complex by using a PET/CT or SPECT/CT device. The diagnosis of a cancer is preferably performed in combination of, for example, an immunochemistry tissue staining technique, an imaging diagnostic method using X-rays, or the like.

Specific examples of the subject (patient) to which the complex of the present invention is administered for the diagnosis of a cancer include healthy individuals, subjects (patients) suspected to be suffering from cancer, subjects (patients) already proved to have cancer, and subjects (patients) suspected to have recurrence of cancer after treatment of the cancer.

In the diagnosis of a cancer by using the complex of the present invention, a subject (patient) in which a cancer tissue or a cancer cell is detected in their body can be targeted for treatment by surgical treatment, radiation therapy, and/or chemotherapy. The applied surgical treatment and/or chemotherapy are not particularly limited; however, since the complex of the present invention comprises an anti-CAPRIN-1 antibody as a component, treatment using a pharmaceutical composition comprising an anti-CAPRIN-1 antibody as an active ingredient is preferably applied.

The pharmaceutical composition comprising the complex of the present invention as an active ingredient can be formulated by a method known to those skilled in the art. For example, the pharmaceutical composition can be parenterally used in the form of an injectable agent of: an aseptic solution in water or a pharmaceutically acceptable non-water liquid; or a suspension liquid.

For formulation, the complex of the present invention may be combined with a pharmaceutically acceptable carrier, a medium, or an additive, specifically sterilized water, physiological saline, an isotonic liquid, a buffer agent (buffer or the like), plant oil, oil-based liquid, an antioxidant, a solubilizer, an emulsifier, a suspending agent, a surfactant, a stabilizer, an aromatic agent, an excipient, a binder, or the like as appropriate, and may be preferably mixed with such a component in a unit dosage form required for a generally acceptable pharmaceutical practice. The amount of an active ingredient in such a formulation is determined such that an appropriate dosage within an indicated range can be achieved.

For example, a lyophilized product can be formulated by blending the complex of the present invention with an optional salt, a surfactant, a buffer agent, a sugar, and an antifreeze (including some sugars) and lyophilizing the resultant.

A sterilized composition for injection can be prepared in accordance with general formulation practice using a vehicle such as distilled water for injection. An aqueous solution for injection purposes includes, for example, physiological saline or isotonic solutions comprising glucose and other adjuvants such as D-sorbitol, D-mannose, D-mannitol, and sodium chloride. Such solution may be used with an appropriate solubilizer including, for example, alcohols such as ethanol and polyalcohol, such as propylene glycol, polyethylene glycol, or nonionic surfactants such as polysorbate 80 (TM) and HCO-60. Oil-based liquid includes, for example, sesame oil or soybean oil. Such oil-based liquid may be used in combination with benzyl benzoate or benzyl alcohol as a solubilizer. In addition, it may be mixed with a buffering agent such as a phosphate buffer or a sodium acetate buffer, a soothing agent such as procaine hydrochloride, a stabilizer such as benzyl alcohol or phenol, or an antioxidant. In general, a formulated injection is filled into an adequate ampule.

The administration is preferably parenteral administration. Specific examples of its dosage forms include injections, intranasal administration preparations, transpulmonary administration preparations, and percutaneous administration preparations. For example, injections can be systemically or locally administered via intravenous injection, intramuscular injection, intraperitoneal injection, subcutaneous injection, or intratumoral injection.

The dose and administration method can be appropriately determined depending on, for example, the age, weight, gender, and symptom of a patient. The dose of a pharmaceutical composition comprising an antibody or a polynucleotide encoding an antibody can be selected within a range of, for example, 0.0001 mg to 1000 mg per kg of body weight per dose. For example, a dose of 0.5 mg, 1 mg, 2 mg, 3 mg, 5 mg, 10 mg, 20 mg, 50 mg, 75 mg, 100 mg, 200 mg, 500 mg, or 1000 mg per kg of body weight per dose can be selected. Alternatively, the dose can be selected within a range of, for example, 0.001 to 100000 mg per patient's body; however, it is not necessarily limited thereto. Alternatively, the dose of the pharmaceutical composition may be determined based on the amount of radiation. The amount can be selected within a range of, for example, 10 KBq to 10 GBq per patient per dose; however, it is not necessarily limited thereto.

Examples

The present invention is specifically described below with reference to Examples. However, the scope of the present invention is not limited to these specific examples.

(Example 1) Anti-CAPRIN-1 Polyclonal Antibody

The anti-CAPRIN-1 polyclonal antibody used in the complex of the present invention has immunological reactivity with the CAPRIN-1 protein. 1 mg of a human CAPRIN-1 recombinant protein shown in SEQ ID NO: 2 was produced according to Example 3 in WO2010/016526 and mixed with an incomplete Freund's adjuvant (IFA) solution in an amount equivalent to the recombinant protein. The mixture was subcutaneously administered to a rabbit four times every 2 weeks. Subsequently, blood was collected to obtain an antiserum containing a polyclonal antibody. The obtained antiserum was purified using a protein G carrier (GE Healthcare Bio-Sciences), and then a polyclonal antibody against the CAPRIN-1 protein (anti-CAPRIN-1 polyclonal antibody #1) was obtained. Additionally, an antibody obtained by purifying the serum of a rabbit, to which no antigen was administered, by using a protein G carrier in a manner similar to the manner described above was used as a rabbit control antibody.

Following polyclonal antibodies #2 to #6 against the partial polypeptides of CAPRIN-1 were obtained by methods similar to the above-described method of producing the polyclonal antibody against the CAPRIN-1 protein.

Anti-CAPRIN-1 polyclonal antibody #2 against partial polypeptide shown in SEQ ID NO: 37 described in WO2011/096528 (SEQ ID NO: 31 in the present specification);
Anti-CAPRIN-1 polyclonal antibody #3 against partial polypeptide shown in SEQ ID NO: 5 described in WO2013/018894 (SEQ ID NO: 32 in the present specification);
Anti-CAPRIN-1 polyclonal antibody #4 against partial polypeptide shown in SEQ ID NO: 5 described in WO2013/125654 (SEQ ID NO: 33 in the present specification);
Anti-CAPRIN-1 polyclonal antibody #5 against partial polypeptide shown in SEQ ID NO: 37 described in WO2011/096533 (SEQ ID NO: 34 in the present specification); and
Anti-CAPRIN-1 polyclonal antibody #6 against partial polypeptide shown in SEQ ID NO: 37 described in WO2011/096534 (SEQ ID NO: 35 in the present specification).

(Example 2) Anti-CAPRIN-1 Monoclonal Antibody

The following anti-CAPRIN-1 monoclonal antibodies were used as anti-CAPRIN-1 monoclonal antibodies used in the complex of the present invention.

First, the following anti-CAPRIN-1 monoclonal was prepared.

A monoclonal antibody against CAPRIN-1 obtained by a method described in WO2011/096528, wherein CDR1 to CDR3 of a heavy-chain variable region consist of the amino acid sequences of SEQ ID NOs: 36, 37, and 38, respectively, and CDR1 to CDR3 of a light-chain variable region consist of SEQ ID NOs: 40, 41, and 42, respectively.

A monoclonal antibody against CAPRIN-1 obtained by a method described in WO2015/020212, wherein CDR1 to CDR3 of a heavy-chain variable region consist of the amino acid sequences of SEQ ID NOs: 44, 45, and 46, respectively, and CDR1 to CDR3 of a light-chain variable region consist of SEQ ID NOs: 48, 49, and 50, respectively.

A monoclonal antibody against CAPRIN-1 obtained by a method described in WO2011/096519, wherein CDR1 to CDR3 of a heavy-chain variable region consist of the amino acid sequences of SEQ ID NOs: 52, 53, and 54, respectively, and CDR1 to CDR3 of a light-chain variable region consist of SEQ ID NOs: 56, 57, and 58, respectively.

A monoclonal antibody against CAPRIN-1 obtained by a method described in WO2013/125654, wherein CDR1 to CDR3 of a heavy-chain variable region consist of the amino acid sequences of SEQ ID NOs: 60, 61, and 62, respectively, and CDR1 to CDR3 of a light-chain variable region consist of SEQ ID NOs: 64, 65, and 66, respectively.

A monoclonal antibody against CAPRIN-1 obtained by a method described in WO2011/096517, the antibody comprising: the amino acid sequence of a heavy-chain variable region shown in the amino acid sequence of SEQ ID NO: 68; and the amino acid sequence of a light-chain variable region shown in the amino acid sequence of SEQ ID NO: 69.

The following monoclonal antibodies against CAPRIN-1 obtained by a method described in WO2011/096528:

an antibody comprising the amino acid sequence of a heavy-chain variable region shown in the amino acid sequence of SEQ ID NO: 70 and the amino acid sequence of a light-chain variable region shown in the amino acid sequence of SEQ ID NO: 71;
an antibody comprising the amino acid sequence of a heavy-chain variable region shown in the amino acid sequence of SEQ ID NO: 72 and the amino acid sequence of a light-chain variable region shown in the amino acid sequence of SEQ ID NO: 73;
an antibody comprising the amino acid sequence of a heavy-chain variable region shown in the amino acid sequence of SEQ ID NO: 74 and the amino acid sequence of a light-chain variable region shown in the amino acid sequence of SEQ ID NO: 75;
an antibody comprising the amino acid sequence of a heavy-chain variable region shown in the amino acid sequence of SEQ ID NO: 76 and the amino acid sequence of a light-chain variable region shown in the amino acid sequence of SEQ ID NO: 77; and
an antibody comprising the amino acid sequence of a heavy-chain variable region shown in the amino acid sequence of SEQ ID NO: 78 and the amino acid sequence of a light-chain variable region shown in the amino acid sequence of SEQ ID NO: 79.

The following monoclonal antibodies against CAPRIN-1 obtained by a method described in WO2011/096533:

an antibody comprising the amino acid sequence of a heavy-chain variable region shown in the amino acid sequence of SEQ ID NO: 80 and the amino acid sequence of a light-chain variable region shown in the amino acid sequence of SEQ ID NO: 81; and
an antibody comprising the amino acid sequence of a heavy-chain variable region shown in the amino acid sequence of SEQ ID NO: 82 and the amino acid sequence of a light-chain variable region shown in the amino acid sequence of SEQ ID NO: 83.

The following monoclonal antibodies against CAPRIN-1 obtained by a method described in WO2011/096534:

an antibody comprising the amino acid sequence of a heavy-chain variable region shown in the amino acid sequence of SEQ ID NO: 84 and the amino acid sequence of a light-chain variable region shown in the amino acid sequence of SEQ ID NO: 85; and
an antibody comprising the amino acid sequence of a heavy-chain variable region shown in the amino acid sequence of SEQ ID NO: 86 and the amino acid sequence of a light-chain variable region shown in the amino acid sequence of SEQ ID NO: 87.

The following monoclonal antibodies against CAPRIN-1 obtained by a method described in WO2010/016526:

an antibody comprising the amino acid sequence of a heavy-chain variable region shown in the amino acid sequence of SEQ ID NO: 88 and the amino acid sequence of a light-chain variable region shown in the amino acid sequence of SEQ ID NO: 89;
an antibody comprising the amino acid sequence of a heavy-chain variable region shown in the amino acid sequence of SEQ ID NO: 90 and the amino acid sequence of a light-chain variable region shown in the amino acid sequence of SEQ ID NO: 91;
an antibody comprising the amino acid sequence of a heavy-chain variable region shown in the amino acid sequence of SEQ ID NO: 92 and the amino acid sequence of a light-chain variable region shown in the amino acid sequence of SEQ ID NO: 93;
an antibody comprising the amino acid sequence of a heavy-chain variable region shown in the amino acid sequence of SEQ ID NO: 94 and the amino acid sequence of a light-chain variable region shown in the amino acid sequence of SEQ ID NO: 95;
an antibody comprising the amino acid sequence of a heavy-chain variable region shown in the amino acid sequence of SEQ ID NO: 96 and the amino acid sequence of a light-chain variable region shown in the amino acid sequence of SEQ ID NO: 97;
an antibody comprising the amino acid sequence of a heavy-chain variable region shown in the amino acid sequence of SEQ ID NO: 98 and the amino acid sequence of a light-chain variable region shown in the amino acid sequence of SEQ ID NO: 99; and
an antibody comprising the amino acid sequence of a heavy-chain variable region shown in the amino acid sequence of SEQ ID NO: 100 and the amino acid sequence of a light-chain variable region shown in the amino acid sequence of SEQ ID NO: 101.

The following monoclonal antibodies against CAPRIN-1 obtained by a method described in WO2013/018894:

an antibody comprising the amino acid sequence of a heavy-chain variable region shown in the amino acid sequence of SEQ ID NO: 102 and the amino acid sequence of a light-chain variable region shown in the amino acid sequence of SEQ ID NO: 103; and
an antibody comprising the amino acid sequence of a heavy-chain variable region shown in the amino acid sequence of SEQ ID NO: 104 and the amino acid sequence of a light-chain variable region shown in the amino acid sequence of SEQ ID NO: 105.

A monoclonal antibody against CAPRIN-1 obtained by a method described in WO2013/018892, the antibody comprising the amino acid sequence of a heavy-chain variable region shown in the amino acid sequence of SEQ ID NO: 106 and the amino acid sequence of a light-chain variable region shown in the amino acid sequence of SEQ ID NO: 107.
A monoclonal antibody against CAPRIN-1 obtained by a method described in WO2013/018891, the antibody comprising the amino acid sequence of a heavy-chain variable region shown in the amino acid sequence of SEQ ID NO: 108 and the amino acid sequence of a light-chain variable region shown in the amino acid sequence of SEQ ID NO: 109.
A monoclonal antibody against CAPRIN-1 obtained by a method described in WO2013/018889, the antibody comprising the amino acid sequence of a heavy-chain variable region shown in the amino acid sequence of SEQ ID NO: 110 and the amino acid sequence of a light-chain variable region shown in the amino acid sequence of SEQ ID NO: 111.
A monoclonal antibody against CAPRIN-1 obtained by a method described in WO2013/018883, the antibody comprising the amino acid sequence of a heavy-chain variable region shown in the amino acid sequence of SEQ ID NO: 112 and the amino acid sequence of a light-chain variable region shown in the amino acid sequence of SEQ ID NO: 113.
A monoclonal antibody against CAPRIN-1 obtained by a method described in WO2013/125636, the antibody comprising the amino acid sequence of a heavy-chain variable region shown in the amino acid sequence of SEQ ID NO:

114 and the amino acid sequence of a light-chain variable region shown in the amino acid sequence of SEQ ID NO: 115.

The following monoclonal antibodies against CAPRIN-1 obtained by a method described in WO2013/125654:

an antibody comprising the amino acid sequence of a heavy-chain variable region shown in the amino acid sequence of SEQ ID NO: 116 and the amino acid sequence of a light-chain variable region shown in the amino acid sequence of SEQ ID NO: 117; and

an antibody comprising the amino acid sequence of a heavy-chain variable region shown in the amino acid sequence of SEQ ID NO: 118 and the amino acid sequence of a light-chain variable region shown in the amino acid sequence of SEQ ID NO: 119.

A monoclonal antibody against CAPRIN-1 obtained by a method described in WO2013/125630, the antibody comprising the amino acid sequence of a heavy-chain variable region shown in the amino acid sequence of SEQ ID NO: 120 and the amino acid sequence of a light-chain variable region shown in the amino acid sequence of SEQ ID NO: 121.

The following monoclonal antibodies against CAPRIN-1 obtained by a method described in WO2015/020212:

an antibody comprising the amino acid sequence of a heavy-chain variable region shown in the amino acid sequence of SEQ ID NO: 122 and the amino acid sequence of a light-chain variable region shown in the amino acid sequence of SEQ ID NO: 123;

an antibody comprising the amino acid sequence of a heavy-chain variable region shown in the amino acid sequence of SEQ ID NO: 124 and the amino acid sequence of a light-chain variable region shown in the amino acid sequence of SEQ ID NO: 125;

an antibody comprising the amino acid sequence of a heavy-chain variable region shown in the amino acid sequence of SEQ ID NO: 126 and the amino acid sequence of a light-chain variable region shown in the amino acid sequence of SEQ ID NO: 127;

an antibody comprising the amino acid sequence of a heavy-chain variable region shown in the amino acid sequence of SEQ ID NO: 128 and the amino acid sequence of a light-chain variable region shown in the amino acid sequence of SEQ ID NO: 129;

an antibody comprising the amino acid sequence of a heavy-chain variable region shown in the amino acid sequence of SEQ ID NO: 130 and the amino acid sequence of a light-chain variable region shown in the amino acid sequence of SEQ ID NO: 131; and

an antibody comprising the amino acid sequence of a heavy-chain variable region shown in the amino acid sequence of SEQ ID NO: 132 and the amino acid sequence of a light-chain variable region shown in the amino acid sequence of SEQ ID NO: 133.

Then, a humanized antibody was produced from the anti-CAPRIN-1 monoclonal antibody described above.

A nucleotide sequence was designed so that CDR1 to CDR3 of a heavy-chain variable region consist of the amino acid sequences of SEQ ID NOs: 36, 37, and 38, respectively, and framework regions are able to express heavy-chain variable regions comprising human antibody sequences. The nucleotide sequence was inserted into a vector for mammalian expression having an insert of a human IgG1 heavy-chain constant region. Likewise, a nucleotide sequence was designed so that CDR1 to CDR3 of a light-chain variable region consist of SEQ ID NO: 40, 41, and 42, respectively, and framework regions are able to express light-chain variable regions comprising human antibody sequences. The nucleotide sequence was inserted into a vector for mammalian expression having an insert of a human IgG1 light-chain constant region. The two recombinant expression vectors described above were introduced into a mammalian cell according to a usual method, to obtain a culture supernatant containing a humanized monoclonal antibody #1 (humanized antibody #1) against CAPRIN-1 in which CDR1 to CDR3 of a heavy-chain variable region consist of the amino acid sequences of SEQ ID NOs: 36, 37, and 38, respectively, and CDR1 to CDR3 of a light-chain variable region consist of SEQ ID NOs: 40, 41, and 42, respectively.

Likewise, culture supernatants containing the following humanized anti-CAPRIN-1 monoclonal antibody #2 to #4 (humanized antibodies #2 to #4) were obtained.

The humanized anti-CAPRIN-1 monoclonal antibody #2 (humanized antibody #2) in which CDR1 to CDR3 of a heavy-chain variable region consist of the amino acid sequences of SEQ ID NOs: 44, 45, and 46, and CDR1 to CDR3 of a light-chain variable region consist of the amino acid sequences of SEQ ID NOs: 48, 49, and 50, respectively.

The humanized anti-CAPRIN-1 monoclonal antibody #3 (humanized antibody #3) in which CDR1 to CDR3 of a heavy-chain variable region consist of the amino acid sequences of SEQ ID NOs: 52, 53, and 54, respectively, and CDR1 to CDR3 of a light-chain variable region consist of SEQ ID NOs: 56, 57, and 58, respectively.

The humanized anti-CAPRIN-1 monoclonal antibody #4 (humanized antibody #4) in which CDR1 to CDR3 of a heavy-chain variable region consist of the amino acid sequences of SEQ ID NOs: 60, 61, and 62, respectively, and CDR1 to CDR3 of a light-chain variable region consist of SEQ ID NOs: 64, 65, and 66, respectively.

Likewise, culture supernatants containing the following humanized anti-CAPRIN-1 monoclonal antibody #9 to #41 (humanized antibodies # 9 to #41) were obtained.

The humanized monoclonal antibody #9 (humanized antibody #9) comprising the amino acid sequence of a heavy-chain variable region shown in the amino acid sequence of SEQ ID NO: 68 and the amino acid sequence of a light-chain variable region shown in the amino acid sequence of SEQ ID NO: 69.

The humanized monoclonal antibody #10 (humanized antibody #10) comprising the amino acid sequence of a heavy-chain variable region shown in the amino acid sequence of SEQ ID NO: 70 and the amino acid sequence of a light-chain variable region shown in the amino acid sequence of SEQ ID NO: 71.

The humanized monoclonal antibody #11 (humanized antibody #11) comprising the amino acid sequence of a heavy-chain variable region shown in the amino acid sequence of SEQ ID NO: 72 and the amino acid sequence of a light-chain variable region shown in the amino acid sequence of SEQ ID NO: 73.

The humanized monoclonal antibody #12 (humanized antibody #12) comprising the amino acid sequence of a heavy-chain variable region shown in the amino acid sequence of SEQ ID NO: 74 and the amino acid sequence of a light-chain variable region shown in the amino acid sequence of SEQ ID NO: 75.

The humanized monoclonal antibody #13 (humanized antibody #13) comprising the amino acid sequence of a heavy-chain variable region shown in the amino acid sequence of SEQ ID NO: 76 and the amino acid sequence of a light-chain variable region shown in the amino acid sequence of SEQ ID NO: 77.

The humanized monoclonal antibody #14 (humanized antibody #14) comprising the amino acid sequence of a heavy-chain variable region shown in the amino acid sequence of SEQ ID NO: 78 and the amino acid sequence of a light-chain variable region shown in the amino acid sequence of SEQ ID NO: 79.

The humanized monoclonal antibody #15 (humanized antibody #15) comprising the amino acid sequence of a heavy-chain variable region shown in the amino acid sequence of SEQ ID NO: 80 and the amino acid sequence of a light-chain variable region shown in the amino acid sequence of SEQ ID NO: 81.

The humanized monoclonal antibody #16 (humanized antibody #16) comprising the amino acid sequence of a heavy-chain variable region shown in the amino acid sequence of SEQ ID NO: 82 and the amino acid sequence of a light-chain variable region shown in the amino acid sequence of SEQ ID NO: 83.

The humanized monoclonal antibody #17 (humanized antibody #17) comprising the amino acid sequence of a heavy-chain variable region shown in the amino acid sequence of SEQ ID NO: 84 and the amino acid sequence of a light-chain variable region shown in the amino acid sequence of SEQ ID NO: 85.

The humanized monoclonal antibody #18 (humanized antibody #18) comprising the amino acid sequence of a heavy-chain variable region shown in the amino acid sequence of SEQ ID NO: 86 and the amino acid sequence of a light-chain variable region shown in the amino acid sequence of SEQ ID NO: 87.

The antibody humanized monoclonal antibody #19 (humanized antibody #19) comprising the amino acid sequence of a heavy-chain variable region shown in the amino acid sequence of SEQ ID NO: 88 and the amino acid sequence of a light-chain variable region shown in the amino acid sequence of SEQ ID NO: 89.

The humanized monoclonal antibody #20 (humanized antibody #20) comprising the amino acid sequence of a heavy-chain variable region shown in the amino acid sequence of SEQ ID NO: 90 and the amino acid sequence of a light-chain variable region shown in the amino acid sequence of SEQ ID NO: 91.

The humanized monoclonal antibody #21 (humanized antibody #21) comprising the amino acid sequence of a heavy-chain variable region shown in the amino acid sequence of SEQ ID NO: 92 and the amino acid sequence of a light-chain variable region shown in the amino acid sequence of SEQ ID NO: 93.

The humanized monoclonal antibody #22 (humanized antibody #22) comprising the amino acid sequence of a heavy-chain variable region shown in the amino acid sequence of SEQ ID NO: 94 and the amino acid sequence of a light-chain variable region shown in the amino acid sequence of SEQ ID NO: 95.

The humanized monoclonal antibody #23 (humanized antibody #23) comprising the amino acid sequence of a heavy-chain variable region shown in the amino acid sequence of SEQ ID NO: 96 and the amino acid sequence of a light-chain variable region shown in the amino acid sequence of SEQ ID NO: 97.

The humanized monoclonal antibody #24 (humanized antibody #24) comprising the amino acid sequence of a heavy-chain variable region shown in the amino acid sequence of SEQ ID NO: 98 and the amino acid sequence of a light-chain variable region shown in the amino acid sequence of SEQ ID NO: 99.

The humanized monoclonal antibody #25 (humanized antibody #25) comprising the amino acid sequence of a heavy-chain variable region shown in the amino acid sequence of SEQ ID NO: 100 and the amino acid sequence of a light-chain variable region shown in the amino acid sequence of SEQ ID NO: 101.

The humanized monoclonal antibody #26 (humanized antibody #26) comprising the amino acid sequence of a heavy-chain variable region shown in the amino acid sequence of SEQ ID NO: 102 and the amino acid sequence of a light-chain variable region shown in the amino acid sequence of SEQ ID NO: 103.

The humanized monoclonal antibody #27 (humanized antibody #27) comprising the amino acid sequence of a heavy-chain variable region shown in the amino acid sequence of SEQ ID NO: 104 and the amino acid sequence of a light-chain variable region shown in the amino acid sequence of SEQ ID NO: 105.

The humanized monoclonal antibody #28 (humanized antibody #28) comprising the amino acid sequence of a heavy-chain variable region shown in the amino acid sequence of SEQ ID NO: 106 and the amino acid sequence of a light-chain variable region shown in the amino acid sequence of SEQ ID NO: 107.

The humanized monoclonal antibody #29 (humanized antibody #29) comprising the amino acid sequence of a heavy-chain variable region shown in the amino acid sequence of SEQ ID NO: 108 and the amino acid sequence of a light-chain variable region shown in the amino acid sequence of SEQ ID NO: 109.

The humanized monoclonal antibody #30 (humanized antibody #30) comprising the amino acid sequence of a heavy-chain variable region shown in the amino acid sequence of SEQ ID NO: 110 and the amino acid sequence of a light-chain variable region shown in the amino acid sequence of SEQ ID NO: 111.

The humanized monoclonal antibody #31 (humanized antibody #31) comprising the amino acid sequence of a heavy-chain variable region shown in the amino acid sequence of SEQ ID NO: 112 and the amino acid sequence of a light-chain variable region shown in the amino acid sequence of SEQ ID NO: 113.

The humanized monoclonal antibody #32 (humanized antibody #32) comprising the amino acid sequence of a heavy-chain variable region shown in the amino acid sequence of SEQ ID NO: 114 and the amino acid sequence of a light-chain variable region shown in the amino acid sequence of SEQ ID NO: 115.

The humanized monoclonal antibody #33 (humanized antibody #33) comprising the amino acid sequence of a heavy-chain variable region shown in the amino acid sequence of SEQ ID NO: 116 and the amino acid sequence of a light-chain variable region shown in the amino acid sequence of SEQ ID NO: 117.

The humanized monoclonal antibody #34 (humanized antibody #34) comprising the amino acid sequence of a heavy-chain variable region shown in the amino acid sequence of SEQ ID NO: 118 and the amino acid sequence of a light-chain variable region shown in the amino acid sequence of SEQ ID NO: 119.

The humanized monoclonal antibody #35 (humanized antibody #35) comprising the amino acid sequence of a heavy-chain variable region shown in the amino acid sequence of SEQ ID NO: 120 and the amino acid sequence of a light-chain variable region shown in the amino acid sequence of SEQ ID NO: 121.

The humanized monoclonal antibody #36 (humanized antibody #36) comprising the amino acid sequence of a heavy-chain variable region shown in the amino acid sequence of SEQ ID NO: 122 and the amino acid sequence of a light-chain variable region shown in the amino acid sequence of SEQ ID NO: 123.

The humanized monoclonal antibody #37 (humanized antibody #37) comprising the amino acid sequence of a heavy-chain variable region shown in the amino acid sequence of SEQ ID NO: 124 and the amino acid sequence of a light-chain variable region shown in the amino acid sequence of SEQ ID NO: 125.

The humanized monoclonal antibody #38 (humanized antibody #38) comprising the amino acid sequence of a heavy-chain variable region shown in the amino acid sequence of SEQ ID NO: 126 and the amino acid sequence of a light-chain variable region shown in the amino acid sequence of SEQ ID NO: 127.

The humanized monoclonal antibody #39 (humanized antibody #39) comprising the amino acid sequence of a heavy-chain variable region shown in the amino acid sequence of SEQ ID NO: 128 and the amino acid sequence of a light-chain variable region shown in the amino acid sequence of SEQ ID NO: 129.

The humanized monoclonal antibody #40 (humanized antibody #40) comprising the amino acid sequence of a heavy-chain variable region shown in the amino acid sequence of SEQ ID NO: 130 and the amino acid sequence of a light-chain variable region shown in the amino acid sequence of SEQ ID NO: 131.

The humanized monoclonal antibody #41 (humanized antibody #41) comprising the amino acid sequence of a heavy-chain variable region shown in the amino acid sequence of SEQ ID NO: 132 and the amino acid sequence of a light-chain variable region shown in the amino acid sequence of SEQ ID NO: 133.

Additionally, a mammalian expression vector having insertion of the heavy-chain constant region of human IgG1, in which serine (Ser) at amino acid position 239 in the EU numbering was substituted with aspartic acid (Asp) and isoleucine (Ile) at amino acid position 332 in the EU numbering was substituted with glutamic acid (Glu) was used. Then, culture supernatants containing, respectively, monoclonal antibody #5 to #8 (humanized antibodies #5 to #8) against humanized CAPRIN-1 consisting of heavy chain full-length amino acid sequence comprising human IgG1 heavy-chain constant regions in which, in the heavy-chain constant region of each of the humanized antibodies #1 to #4, serine (Ser) at amino acid position 239 in the EU numbering was substituted with aspartic acid (Asp) and isoleucine (Ile) at amino acid position 332 in the EU numbering was substituted with glutamic acid (Glu), and of the light chain full-length amino acid sequence same as those of the humanized antibodies #1 to #4 were obtained.

Likewise, culture supernatants containing, respectively, humanized anti-CAPRIN-1 monoclonal antibody #42 to #74 (humanized antibodies #42 to #74), wherein, in the heavy-chain variable region of each of the humanized antibodies #9 to #41 produced as described above, serine (Ser) at amino acid position 239 in the EU numbering was substituted with aspartic acid (Asp), and isoleucine (Ile) at amino acid position 332 in the EU numbering was substituted with glutamic acid (Glu), were obtained.

The obtained culture supernatants containing the humanized antibody #1 to #74 were purified using Hitrap Protein A Sepharose FF (manufactured by GE Healthcare Bio-Sciences) according to a usual method, replaced with PBS (-), and filtered through a 0.22 $\mu$m filter (manufactured by Millipore) to prepare the filtrate.

(Example 3) Binding between Anti-CAPRIN-1 Antibody and Chelating Agent (DOTA)

The complex of DOTA and each of the anti-CAPRIN-1 polyclonal antibodies #1 to #6 described in Example 1 and the humanized antibodies #1 to #74 described in Example 2 was produced according to the following method.

The solution containing each antibody was replaced with 0.1 M phosphate buffer (pH 8.0) treated with Chelex (registered trademark) 100 in order to remove metal ions in the solution in advance by using AmiconUltra-50K according to a usual method. The solution containing the antibody was adjusted to 7.5 mg/ml.

DOTA-NHS-ester (DOTA-NHS-ester/CFCOOH/HPF6 (C084, CheMatech)) was adjusted to 10 mg/ml by using distilled water treated with Chelex (registered trademark) 100 in order to remove metal ions in the solution in advance, and a DOTA-NHS-ester solution was obtained. The DOTA-NHS-ester solution was added to each antibody so as to achieve a molar ratio of 10. The resultant was allowed to react at room temperature for 4 hours while shaking the resultant. Then, the resultant was allowed to react at 4°C for about 18 hours to obtain a solution containing the complex of the antibody and DOTA. The solution containing the complex was replaced with DPBS (-) treated with Chelex100 (registered trademark) in order to remove metal ions in the solution in advance by using AmiconUltra-50K, adjusted to 10 mg/ml, and then filtered using a 0.22 $\mu$m filtration membrane for sterilization. Solutions containing the complex of DOTA and the respective anti-CAPRIN-1 polyclonal antibodies #1 to #6 (polyclonal antibody complexes #1 to #6) and the complex of DOTA and the respective humanized antibodies #1 to #74 (humanized antibody complexes #1 to #74) were obtained.

As a result of measurement of the number of DOTAs bound per molecule of anti-CAPRIN-1 antibody by mass spectrometry for the complexes produced as described above, about two to four DOTAs were bound per antibody molecule.

(Example 4) Specific Reactivity of Polyclonal Antibody Complex and Humanized Antibody Complex with CAPRIN-1 and CAPRIN-1-Expressing Cancer Cell

The specific reactivity of the complexes produced in Example 3 with CAPRIN-1 protein was evaluated. Further, whether the complex exhibits reactivity with the cell membrane surfaces of human cancer cells and mouse cancer cells expressing the CAPRIN-1 protein on the cell membrane surfaces was evaluated.

The specific reactivity with the CAPRIN-1 protein was confirmed by using ELISA. CAPRIN-1 protein solution (2 $\mu$g/mL) was added at 100 $\mu$L per well of 96-well plates, and left to stand at 4°C for about 18 hours. After each well was washed three times with PBS-T, a DPBS solution containing 2% Bovine Serum Albumin (BSA) was added at 200 $\mu$L per well, and left to stand at room temperature for 1 hour. The solution was removed, and each well was washed three times with 300 $\mu$L of PBS-T per well. Then, each of solutions containing the polyclonal antibody complex #1 to #6, the humanized antibody complex #1 to #74, and respective positive control antibodies (polyclonal antibodies #1 to #6 and humanized antibodies #1 to #74) was added at 100 $\mu$L per well, and left to stand at room temperature for 1 hour. After each well was washed three times with PBS-T, an HRP-labeled anti-rabbit IgG antibody or an HRP-labeled anti-human IgG antibody (Abcam) diluted 5000-fold with DPBS was added at 50 $\mu$L per well, and left to stand at room temperature for 30 minutes. Each well was washed three times with PBS-T, a TMB substrate solution was added at 50 $\mu$L per well, and then left to stand for 3 to 5 minutes to perform a color reaction. After color development, Stop Solution (Theromo Scientific) was added at 50 $\mu$L per well to stop the reaction. An absorbance values at 450 nm and 630 nm were measured using an absorption spectrometer. As a result, the polyclonal antibody complexes #1 to #6 and the humanized antibody complexes #1 to #74 exhibited absorbance values equivalent to the absorbance values of the respective positive control antibodies (the polyclonal antibodies #2 to #6 and the humanized antibodies #1 to #74). The complexes were found to specifically react with the CAPRIN-1 protein.

Then, the reactivity with a cell membrane surface of a cancer cell expressing CAPRIN-1 on the cell membrane surface thereof was confirmed by a flow cytometry method. To each of $5 \times 10^4$ human colorectal cancer cells HT29 (ATCC) and mouse breast cancer cells 4T1 (ATCC), 50 $\mu$L of solution containing each complex and control antibody was added, respectively. The resultant was left to stand at 4°C for 30 minutes. After washing with DPBS, Alexa488-labeled anti-rabbit IgG antibody or Alexa488-labeled anti-human IgG antibody diluted with DPBS (-) containing 1% FBS (1% FBS-DPBS (-)) at 10 $\mu$g/mL was added, and the resultant was left to stand at 4°C for 30 minutes. After washing with 1% FBS-DPBS (-), reaction with BD Horizon Fixable Viability Stain 450 (FVS450, BD Biosciences) was performed to stain dead cells, and fluorescence intensity was then measured by LSR Fortessa (trademark) (BD Biosciences). As a result, the fluorescence intensity of each of the polyclonal antibody complexes #1 to #6 and the humanized antibody complexes #1 to #74 was higher than that of a negative control antibody (rabbit IgG antibody or human IgG antibody). That is, each of the polyclonal antibody complexes #1 to #6 and the humanized antibody complexes #1 to #74 was found to strongly react with the cell surfaces of human cancer cells HT29 and mouse cancer cells 4T1 expressing CAPRIN-1.

Likewise, the reactivity of the complexes produced in Example 3 with the following various human cancer cells and mouse cancer cells was confirmed. Breast cancer cells (BT-474), colorectal cancer cells (HT29, HCT116, and Lovo), lung cancer cells (A549), gastric cancer cells (NCI-N87), uterine cancer cells (HEC-1-A), prostate cancer cells (22Rv1), pancreatic cancer cells (Panc10.5), liver cancer cells (HepG2), ovarian cancer cells (MCAS, SKOV3, and TOV112D), renal cancer cells (Caki-2), brain tumor cells (U-87MG), bladder cancer cells (T24, UMUC3), bile duct cancer cells (KKU213), fibrosarcoma cells (HT-1080), esophageal cancer cells (OE33), leukemia cells (OCI-AML5), lymphoma cells (Ramos), gallbladder cancer cells (TGBC14TKB), melanoma cells (Malme-3M), and head and neck cancer cells (FaDu) are human cancer cells confirmed to express CAPRIN-1 gene and to express CAPRIN-1 on the cell membrane surfaces of the cancer cells. Mouse renal cancer cells (Renca) and mouse breast cancer cells (4T1) are confirmed to express CAPRIN-1 gene and to express CAPRIN-1 on the cell membrane surfaces of the cancer cells. The complexes produced in Example 3 had stronger fluorescence intensity against above cancer cells than those of the negative control antibodies confirming strong reactivity with the cell membrane surfaces of the cancer cells expressing CAPRIN-1.

(Example 5) Labeling of Polyclonal Antibody Complex and Humanized Antibody Complex with Radioisotope (111-In)

The polyclonal antibody complexes and humanized antibody complexes produced in Example 3 were labeled to produce a complex labeled with a metal radioisotope 111-In. Each of solutions containing the polyclonal antibody complex #1 to #6 and humanized antibody complex #1 to #74 obtained in Example 3 was adjusted to about 10 mg/ml, and mixed with 0.5 M HEPES buffer (pH $5.3 \pm 0.3$). Then, 111-InCl$_3$ (Curium Pharma) was added to the mixture (mixture volume ratio = 5:10:8), and the resultant was allowed to react at about 38°C for about 30 minutes. Part of the solution after the reaction was collected, and the radiochemical purity thereof was calculated by thin-layer chromatography with 0.1 M citric acid buffer as a developing solvent.

Then, purification was performed using AmiconUltra-50K, to obtain 111-In-labeled polyclonal antibody complexes #1 to #6 and 111-In-labeled humanized antibody complexes #1 to #74 having a radiochemical purity of 95% or more.

(Example 6) Detection of Cancer by Using 111-In-Labeled Polyclonal Antibody Complex and 111-In-Labeled Humanized Antibody Complex

The *in vivo* cancer detection capability of the 111-In-labeled humanized antibody complex #1 produced in Example 5 was evaluated by using cancer-bearing mice obtained by transplanting human-derived cancer cells (human colorectal cancer cells HT29) expressing the CAPRIN-1 protein on cell membrane surfaces into NMRI-Nude mice. $10^7$ human colorectal cancer cells HT29 per mouse were mixed with ECM gel (SIGMA-Aldrich). The mixture was subcutaneously transplanted to form a tumor to produce cancer-bearing mice. Single-dose administration of about 6.5 MBq of the 111-In-labeled humanized antibody complex #1 to the mice bearing cancer HT29 through the tail vein was performed under anesthesia with isoflurane.

In order to detect cancer transplanted in the mice, $\gamma$ rays radiated from the 111-In-labeled humanized antibody complex #1 were detected by using an imaging device NanoSPECT/CT (Bioscan) according to a usual method under anesthesia with isoflurane 72 hours after the administration of the 111-In-labeled humanized antibody complex #1 to the cancer-bearing mice. HiSPECT NG and InVivoScope (IVS) were used for image reconstruction. As a result of evaluation of the detection of the cancers, images on which the 111-In-labeled humanized antibody complex #1 strongly accumulated on the tumor were successfully acquired indicating that the complex can detect the cancers. Likewise, the cancers were similarly detected in the case of the 111-In-labeled polyclonal antibody complexes #1 to #6 and the 111-In-labeled humanized antibody complexes #2 to #74.

Further, the 111-In-labeled humanized antibody complex #1 was administered in a manner similar to the manner described above, and the organs of the HT29 cancer-bearing mice including the blood, the bone, the brain, the digestive tract, the gonad, the uterus and fallopian tube, the heart, the kidney, the liver, the lung, the muscle, the skin, the spleen, and the tumor were collected, respectively, 72 hours after the administration of the complex. The organs were weighed and then, the amount of radiation radiated from each organ was measured using $\gamma$ counter Wizard2 (PerkinElmer, Inc.). The percentage of radiation accumulated in each organ was calculated based on the amount of dosed radiation and the amount of radiation accumulated in each organ and tumor.

As a result, the percentage of radiation accumulated in the HT29 tumor 72 hours after the administration was about 19% based on the weight of the tumor relative to the amount of radiation emitted from the administered complex (100%). The average of the percentages of the amounts of radiation in the various organs of the mice except the tumors was about 6% (5.5% in the case of excluding the liver which is a major metabolic pathway) based on the weight of the organ. The above results revealed that the complex in which the anti-CAPRIN-1 antibody was labeled with 111-In which is one of radioisotopes can detect a cancer. Likewise, similar results were obtained in the 111-In-labeled polyclonal antibody complexes #1 to #6 and the 111-In-labeled humanized antibody complexes #2 to #74.

(Example 7) Labeling of Polyclonal Antibody Complex and Humanized Antibody Complex with Radioisotope (177-Lu)

The polyclonal antibody complexes and humanized antibody complexes produced in Example 3 were labeled to produce a complex labeled with a metal radioisotope 177-Lu. Each of solutions containing the polyclonal antibody complex #1 to #6 and humanized antibody complex #1 to #74 obtained in Example 3 was adjusted to about 10 mg/ml, and mixed with 0.2 M sodium acetate buffer (pH 5.3 to 5.6). Then, $177\text{-}LuCl_3$ solution (ITM Medical Isotopes) was added to the mixture (mixture volume ratio = 20:25:2), and the resultant was allowed to react at about 38°C for about 18 hours. Part of the solution after the reaction was collected, and the radiochemical purity thereof was calculated by thin-layer chromatography with 0.1 M citric acid buffer as a developing solvent by the following usual method. Then, purification was performed using AmiconUltra-50K to obtain 177-Lu-labeled polyclonal antibody complexes #1 to #6 and 177-Lu-labeled humanized antibody complexes #1 to #74 having a radiochemical purity of 95% or more.

(Example 8) Anti-tumor Effect (HT29) of 177-Lu-Labeled Polyclonal Antibody Complex and 177-Lu-Labeled Humanized Antibody Complex on Cancer-Bearing Mouse

The *in vivo* anti-tumor effect of the 177-Lu-labeled humanized antibody complex #1 produced in Example 7 in the cancer-bearing mouse with a human colorectal cancer cell HT29 produced in a method similar to the method in Example 6 was evaluated. Single-dose administration of about 10 MBq of the 177-Lu-labeled humanized antibody complex #1 to mice bearing cancer HT29 through the tail vein was performed. Comparative control groups were a physiological saline treated group (no treatment group), a humanized antibody #1 treated group, a humanized antibody complex #1 without radioisotope labeling treated group, and a 177-Lu-labeled DOTATATE (equivalent to Lutathera (registered trademark) marketed as a therapeutic agent for somatostatin receptor positive cancer) treated group. HT29 expresses a somatostatin receptor which is targeted by 177-Lu-labeled DOTATATE (Biomedicines 2021, 9, 1743).
The sizes of the cancers in the cancer-bearing mice after the administration were measured over time using calipers. The tumor volume was calculated according to a usual method using a calculation expression: (length of major axis of cancer) $\times$ (length of minor axis of cancer)$^2 \times$ 0.5. As a result of the evaluation, the tumor volumes of the mice to which the 177-Lu-labeled humanized antibody complex #1 was administered were about 45% or less on day 31 after the start of the administration relative to the tumor volume of the humanized antibody complex #1 without radioisotope labeling treated group (100%). The tumor volumes of mice to which the complex without radioisotope labeling was administered were equivalent to those of the non-treatment group and the humanized antibody #1 treated group. The tumor volumes of mice to which DOTATATE labeled with 177-Lu was administered were about 80% or more relative to the tumor volume of a mouse to which DOTATATE without radioisotope labeling was administered (100%). The tumor volumes of mice to which DOTATATE without radioisotope labeling were equivalent to those of the non-treatment group. As a result of this evaluation, it was revealed that the 177-Lu-labeled humanized antibody complex #1 exerts a much stronger anti-tumor effect than those of the humanized antibody #1, the humanized antibody complex #1 without radioisotope labeling, and DOTATATE labeled with 177-Lu. Similar results of the 177-Lu-labeled humanized antibody complex #1 were obtained in the 177-Lu-labeled polyclonal antibody complexes #1 to #6, and the 177-Lu-labeled humanized antibody complexes #2 to #74.

(Example 9) Production of Anti-CAPRIN-1 Fab and Anti-CAPRIN-1 F(ab')$_2$

Each solvent of the humanized antibodies #1 to #74 was replaced with Digestion buffer (Thermo Scientific) according to a usual method by using Zeba Spin Desalting Columns 40K MWCO. A Papain solution (1/60 of the amount of antibody) was added to the resultant, and allowed to react at 37°C for 240 minutes. The reaction liquid was centrifuged, and a supernatant was then collected and passed through a 0.22 $\mu$m filter to remove Papain. Then, an unreacted antibody and an Fc fragment were removed using AKTA pure25 (GE Healthcare) to obtain anti-CAPRIN-1 Fab #1 to #74.
Each solvent of the humanized antibodies #1 to #74 was replaced with 0.1 M citric acid buffer (pH 3.5) according to a usual method by using Zeba Spin Desalting Columns 40K MWCO. A pepsin solution (1/90 of the amount of antibody) was added to the resultant, and allowed to react at 37°C for 60 minutes. Then, 1 M Tris-HCl (pH 9.0) was added to stop the reaction. Then, the solvent was replaced with DPSB (-) by a usual method using AmiconUltra-10K, and fractionation was performed using AKTA pure25 (GE Healthcare), to obtain anti-CAPRIN-1F(ab')$_2$ #1 to anti-CAPRIN-1F(ab')$_2$ #74.

(Example 10) Binding between Chelating Agent (DOTA), and Anti-CAPRIN-1 Fab and Anti-CAPRIN-1F(ab')$_2$

The complex of DOTA and each of the anti-CAPRIN-1 Fab #1 to anti-CAPRIN-1 Fab #74 and anti-CAPRIN-1 F(ab')$_2$ #1 to anti-CAPRIN-1 F(ab')$_2$ #74 described in Example 9 was produced according to the following method.
Solutions containing each of anti-CAPRIN-1 Fab and anti-CAPRIN-1 F(ab')$_2$ were replaced with 0.1 M phosphate buffer (pH 8.0) according to a usual method using AmiconUltra-10K. The solutions containing anti-CAPRIN-1 Fab and anti-CAPRIN-1 F(ab')$_2$ were adjusted to 2.5 mg/ml and 5 mg/ml, respectively.

DOTA-NHS-ester (DOTA-NHS-ester/CFCOOH/HPF6 (C084, CheMatech)) was adjusted to 10 mg/ml by using distilled water, to obtain a DOTA-NHS-ester solution. The DOTA-NHS-ester solution was added to each of anti-CAPRIN-1 Fab and anti-CAPRIN-1 F(ab')$_2$ so as to achieve a molar ratio of 10. The resultant was allowed to react at room temperature for 4 hours while shaking the resultant. Then, the resultant was allowed to react at 4°C for about 18 hours, to obtain a solution containing the complex of DOTA, and anti-CAPRIN-1 Fab and anti-CAPRIN-1 F(ab')$_2$. The solution containing the complex was replaced with DPBS (-) by using AmiconUltra-10K. The resultant was adjusted to 10 mg/ml, and then filtered using a 0.22 μm filtration membrane for sterilization, to obtain the solutions containing the complex of DOTA and the respective anti-CAPRIN-1 Fab #1 to anti-CAPRIN-1 Fab #74 (Fab complexes #1 to #74) and the complex of DOTA and the respective anti-CAPRIN-1 F(ab')$_2$ #1 to anti-CAPRIN-1 F(ab')$_2$ #74 (F(ab')$_2$ complexes #1 to #74).

As a result of measurement of the number of DOTAs bound per molecule of each of anti-CAPRIN-1 Fab and anti-CAPRIN-1 F(ab')$_2$ in the complexes produced as described above by mass spectrometry, about one to four DOTAs were bound per molecule of each of anti-CAPRIN-1 Fab and anti-CAPRIN-1 F(ab')$_2$.

(Example 11) Specific Reactivity of Fab Complex and F(ab')$_2$ Complex with CAPRIN-1 and CAPRIN-1-Expressing Cancer Cell

The specific reactivity of the Fab complexes and F(ab')$_2$ complexes produced in Example 10 with CAPRIN-1 protein was evaluated. Further, whether the complex exhibits reactivity with the cell membrane surfaces of human cancer cells and mouse cancer cells expressing the CAPRIN-1 protein on the cell membrane surfaces was evaluated.

The specific reactivity with the CAPRIN-1 protein was confirmed by using ELISA. CAPRIN-1 protein solution (2 μg/mL) was added at 100 μL per well of 96-well plates, and left to stand at 4°C for about 18 hours. After each well was washed three times with PBS-T, 2% Bovine Serum Albumin (BSA) PBS solution was added at 200 μL per well, and left to stand at room temperature for 1 hour. The solution was removed, and each well was washed three times with 300 μL of PBS-T per well. Then, each of solutions containing the Fab complex #1 to #74, F(ab')$_2$ complex #1 to #74, and respective positive controls were added at 100 μL per well, and left to stand at room temperature for 1 hour. After each well was washed three times with PBS-T, an HRP-labeled anti-human IgG (F(ab')$_2$) antibody (Abcam) diluted 5000-fold with DPBS was added at 50 μL per well, and left to stand at room temperature for 30 minutes. Each well was washed three times with PBS-T, a TMB substrate solution was added at 50 μL per well, and then left to stand for 3 to 5 minutes to perform a color reaction. After color development, Stop Solution (Theromo Scientific) was added at 50 μL per well to stop the reaction. An absorbance values at 450 nm and 630 nm were measured using an absorption spectrometer. As a result, the Fab complexes #1 to #74 and the F(ab')$_2$ complexes #1 to #74 exhibited absorbance values equivalent to the absorbance values of the respective positive controls. The complexes were found to specifically react with the CAPRIN-1 protein.

Then, the reactivity with a cell membrane surface of a cancer cell expressing CAPRIN-1 on the cell membrane surface thereof was confirmed by a flow cytometry method. To each of $5 \times 10^4$ human colorectal cancer cells HT29 (obtained from ATCC) and mouse breast cancer cells 4T1 (obtained from ATCC), 50 μL of solution containing Fab complex, F(ab')$_2$ complex, and control antibody was added, respectively. The resultant was left to stand at 4°C for 30 minutes. After washing with DPBS, FITC-labeled anti-IgG (F(ab')$_2$) antibody (Jackson ImmunoResearch) diluted with DPBS (-) containing 1% FBS (1% FBS-DPBS (-)) at 10 μg/mL was added, and the resultant was left to stand at 4°C for 30 minutes. After washing with 1% FBS-DPBS (-), reaction with BD Horizon Fixable Viability Stain 450 (FVS450, BD Biosciences) was performed to stain dead cells, and fluorescence intensity was then measured by LSR Fortessa (BD Biosciences). As a result, the fluorescence intensity of each of the Fab complexes #1 to #74 and the F(ab')$_2$ complexes #1 to #74 was higher than that of a negative control antibody. That is, the Fab complexes #1 to #74 and the F(ab')$_2$ complexes #1 to #74 were found to strongly react with the cell surfaces of human cancer cells HT29 and mouse cancer cells 4T1 expressing CAPRIN-1.

Likewise, the reactivity of the Fab complexes and F(ab')$_2$ complexes produced in Example 10 with the following various human cancer cells and mouse cancer cells was confirmed. Breast cancer cells (BT-474), colorectal cancer cells (HT29, HCT116, and Lovo), lung cancer cells (A549), gastric cancer cells (NCI-N87), uterine cancer cells (HEC-1-A), prostate cancer cells (22Rv1), pancreatic cancer cells (Panc10.5), liver cancer cells (HepG2), ovarian cancer cells (MCAS, SKOV3, and TOV112D), renal cancer cells (Caki-2), brain tumor cells (U-87MG), bladder cancer cells (T24, UMUC3), bile duct cancer cells (KKU213), fibrosarcoma cells (HT-1080), esophageal cancer cells (OE33), leukemia cells (OCI-AML5), lymphoma cells (Ramos), gallbladder cancer cells (TGBC14TKB), melanoma cells (Malme-3M), and head and neck cancer cells (FaDu) are human cancer cells confirmed to express CAPRIN-1 gene and to express CAPRIN-1 on the cell membrane surfaces of the cancer cells. Mouse renal cancer cells (Renca) and mouse breast cancer cells (4T1) are confirmed to express CAPRIN-1 gene and to express CAPRIN-1 on the cell membrane surfaces of the cancer cells. The Fab complexes and the F(ab')$_2$ complexes had stronger fluorescence intensity against above cancer cells than those of the negative controls confirming strong reactivity with the cell membrane surfaces of the cancer cells expressing CAPRIN-1.

(Example 12) Labeling of Fab Complex and F(ab')$_2$ Complex with Radioisotope (111-In)

The Fab complexes and F(ab')$_2$ complexes produced in Example 10 were labeled to produce a complex labeled with a metal radioisotope 111-In. Each of solutions containing the complex obtained in Example 10 was subjected to buffer replacement with an ammonium acetate buffer, then adjusted to about 10 mg/ml, and mixed with a reaction buffer (sodium acetate solution + sodium ascorbate solution + gentisic acid solution, pH 7.4 $\pm$ 0.3). Then, 111-InCl$_3$ solution (Curium Pharma) was added to the mixture (mixture volume ratio = 4.3: 1: 10), and the resultant was allowed to react at about 37°C for 60 minutes. Part of the solution after the reaction was collected, and the radiochemical purity thereof was calculated by thin-layer chromatography with 0.1 M citric acid buffer as a developing solvent.

Then, purification was performed using AmiconUltra-10K, to obtain 111-In-labeled Fab complexes #1 to #74 and 111-In-labeled F(ab')$_2$ complexes #1 to #74 having a radiochemical purity of 95% or more.

(Example 13) Detection of Cancer by Using 111-In-Labeled Fab Complex and 111-In-Labeled F(ab')$_2$ Complex

The *in vivo* cancer detection capability of the 111-In-labeled F(ab')$_2$ complex #1 produced in Example 12 was evaluated by using cancer-bearing mice obtained by transplanting mouse-derived cancer cells (mouse breast cancer cells 4T1) expressing the CAPRIN-1 protein on cell membrane surfaces into Balb/c mice. Cancer-bearing mice were produced by subcutaneously transplanting $10^5$ mouse breast cancer cells 4T1 per mouse to form a tumor. Single-dose administration of about 10 MBq of the 111-In-labeled F(ab')$_2$ complex #1 to the mice bearing cancer 4T1 through the tail vein was performed under anesthesia with isoflurane.

In order to detect cancer transplanted in the mice, $\gamma$ rays radiated from the 111-In-labeled F(ab')$_2$ complex #1 were detected by using an imaging device NanoSPECT/CT (Bioscan) according to a usual method under anesthesia with isoflurane 24 hours after the administration to the cancer-bearing mice. HiSPECT NG and InVivoScope (IVS) were used for image reconstruction. As a result of evaluation of the detection of the cancers, images on which the 111-In-labeled F(ab')$_2$ complex #1 strongly accumulated on the tumor were successfully acquired indicating that the complex can detect the cancers. Likewise, the cancers were similarly detected in the case of the 111-In-labeled Fab complexes #1 to #74 and F(ab')$_2$ complexes #2 to #74.

Further, the 111-In-labeled F(ab')$_2$ complex #1 was administered in a manner similar to the manner described above, and the organs of the 4T1 cancer-bearing mice including the blood, the bone, the brain, the digestive tract, the gonad, the uterus and fallopian tube, the heart, the kidney, the liver, the lung, the muscle, the skin, the spleen, and the tumor were collected, respectively, 24 hours after the administration of the complex. The organs were weighed and then, the amount of radiation radiated from each organ was measured using $\gamma$ counter Wizard2 (PerkinElmer, Inc.). The percentage of radiation accumulated in each organ was calculated based on the amount of dosed radiation and the amount of radiation accumulated in each organ and tumor.

As a result, the percentage of radiation accumulated in the 4T1 tumor 24 hours after the administration was about 13% based on the weight of the tumor relative to the amount of radiation emitted from the administered complex (100%). The average of the percentages of the amounts of radiation in the various organs of the mice except the tumors was 9% (about 4% in the case of excluding the kidney which is a major metabolic pathway) based on the weight of the organ. Similar results were also obtained in the 111-In-labeled Fab complexes #1 to #74 and the F(ab')$_2$ complexes #2 to #74. The above results revealed that the complexes in which the Fab complexes and the F(ab')$_2$ complexes were labeled with 111-In which is one of radioisotopes can detect a cancer.

(Example 14) Labeling of Fab Complex and F(ab')$_2$ Complex with Radioisotope (177-Lu)

The Fab complexes and the F(ab')$_2$ complexes produced in Example 10 were labeled to produce a complex labeled with a metal radioisotope 177-Lu. Each of solutions containing the Fab complex and F(ab')$_2$ complex was adjusted to about 10 mg/ml, and mixed with 0.2 M sodium acetate buffer (pH 5.3 $\pm$ 0.3). Then, 177-LuCl$_3$ solution (ITM Medical Isotopes) was added to the mixture (mixture volume ratio = 20: 25: 2.8), and the resultant was allowed to react at about 38°C for about 18 hours. Part of the solution after the reaction was collected, and the radiochemical purity thereof was calculated by thin-layer chromatography with 0.1 M citric acid buffer as a developing solvent by the following usual method. Then, purification was performed using AmiconUltra-10K to obtain 177-Lu-labeled Fab complexes #1 to #74 and 177-Lu-labeled F(ab')$_2$ complexes #1 to #74 having a radiochemical purity of 95% or more.

(Example 15) Anti-tumor effect (4T1) of 177-Lu-Labeled Fab Complex and 177-Lu-Labeled F(ab')$_2$ Complex on Cancer-Bearing Mouse

The *in vivo* anti-tumor effect of the 177-Lu-labeled Fab complex #1 and 177-Lu-labeled F(ab')$_2$ complex #1 produced in Example 13 in the cancer-bearing mouse with a mouse breast cancer cell 4T1 produced in a method similar to the method in Example 12 was evaluated. Single-dose administration of about 15 MBq of the 177-Lu-labeled Fab complex #1 and 177-Lu-labeled F(ab')$_2$ complex #1 to mice bearing cancer 4T1 through the tail vein was performed. Comparative control groups were a physiological saline treated group (no treatment group), a Fab #1 treated group, an F(ab')$_2$ #1 treated group, a Fab complex #1 without radioisotope labeling treated group, and an F(ab')$_2$ complex #1 without radioisotope labeling treated group.

The sizes of the cancers in the cancer-bearing mice after the administration were measured over time using calipers. The tumor volume was calculated according to a usual method using a calculation expression: (length of major axis of cancer) $\times$ (length of minor axis of cancer)$^2$ $\times$ 0.5. As a result of the evaluation, the tumor volumes of the mice to which the 177-Lu-labeled Fab complex #1 was administered were about 56% or less on day 21 after the start of the administration relative to the tumor volume of the Fab complex #1 without radioisotope labeling treated group (100%). Likewise, the tumor volumes of mice to which the 177-Lu-labeled F(ab')$_2$ complex #1 was administered were about 36% or less relative to the tumor volume of the F(ab')$_2$ complex #1 without radioisotope labeling treated group (100%). The tumor volumes of mice to which Fab, F(ab')$_2$, and the complex without radioisotope labeling were administered were equivalent to those of the non-treatment group. As a result of this evaluation, it was revealed that the 177-Lu-labeled Fab complex #1 and the 177-Lu-labeled F(ab')$_2$ complex #1 exert a much strong anti-tumor effect. Similar results of the 177-Lu-labeled Fab complex #1 and the 177-Lu-labeled F(ab')$_2$ complex #1 were obtained in the 177-Lu-labeled Fab complexes #2 to #74 and the 177-Lu-labeled F(ab')$_2$ complexes #2 to #74. 4T1 expresses a somatostatin receptor (Anti-Cancer Drugs 28 (5): p 489-502, June 2017). Based on the results of the present Example and Example 8, it was estimated that the 177-Lu-labeled Fab complex and the 177-Lu-labeled F(ab')$_2$ complex also have much superior drug efficacy relative to the 177-Lu-labeled DOTATATE (equivalent to Lutathera (registered trademark) marketed as a therapeutic agent for somatostatin receptor positive cancer).

## Claims

1. A complex, wherein a radioisotope(s) and an antibody or a fragment thereof having immunological reactivity with a CAPRIN-1 protein having an amino acid sequence shown in any one of the even numbered SEQ ID NOs: 2 to 30, or an amino acid sequence having 80% or more sequence identity with the amino acid sequence are bound via a chelating agent(s).

2. The complex according to claim 1, wherein the antibody or the fragment thereof has immunological reactivity with a partial polypeptide of the CAPRIN-1 protein, and wherein the partial polypeptide has an amino acid sequence shown in any one of SEQ ID NOs: 31 to 35, 296 to 299, 308, and 309, or an amino acid sequence having 80% or more sequence identity with the amino acid sequence.

3. The complex according to claim 1, wherein the antibody is a monoclonal antibody or a polyclonal antibody.

4. The complex according to claim 1, wherein the antibody or the fragment thereof is any one of the following (A) to (M):

   (A) an antibody or a fragment thereof comprising a heavy-chain variable region comprising complementarity determining regions of SEQ ID NOs: 36, 37, and 38 (CDR1, CDR2, and CDR3, respectively) and a light-chain variable region comprising complementarity determining regions of SEQ ID NOs: 40, 41, and 42 (CDR1, CDR2, and CDR3, respectively), and having an immunological reactivity with the CAPRIN-1 protein;
   (B) an antibody or a fragment thereof comprising a heavy-chain variable region comprising complementarity determining regions of SEQ ID NOs: 44, 45, and 46 (CDR1, CDR2, and CDR3, respectively) and a light-chain variable region comprising complementarity determining regions of SEQ ID NOs: 48, 49, and 50 (CDR1, CDR2, and CDR3, respectively), and having an immunological reactivity with the CAPRIN-1 protein;
   (C) an antibody or a fragment thereof comprising a heavy-chain variable region comprising complementarity determining regions of SEQ ID NOs: 52, 53, and 54 (CDR1, CDR2, and CDR3, respectively) and a light-chain variable region comprising complementarity determining regions of SEQ ID NOs: 56, 57, and 58 (CDR1, CDR2, and CDR3, respectively), and having an immunological reactivity with the CAPRIN-1 protein;
   (D) an antibody or a fragment thereof comprising a heavy-chain variable region comprising complementarity determining regions of SEQ ID NOs: 60, 61, and 62 (CDR1, CDR2, and CDR3, respectively) and a light-chain variable region comprising complementarity determining regions of SEQ ID NOs: 64, 65, and 66 (CDR1, CDR2, and CDR3, respectively), and having an immunological reactivity with the CAPRIN-1 protein;
   (E) an antibody or a fragment thereof comprising a heavy-chain variable region comprising complementarity determining regions of SEQ ID NOs: 170, 171, and 172 (CDR1, CDR2, and CDR3, respectively) and a light-chain variable region comprising complementarity determining regions of SEQ ID NOs: 173, 174, and 175 (CDR1, CDR2, and CDR3, respectively), and having an immunological reactivity with the CAPRIN-1 protein;

(F) an antibody or a fragment thereof comprising a heavy-chain variable region comprising complementarity determining regions of SEQ ID NOs: 176, 177, and 178 (CDR1, CDR2, and CDR3, respectively) and a light-chain variable region comprising complementarity determining regions of SEQ ID NOs: 179, 180, and 181 (CDR1, CDR2, and CDR3, respectively), and having an immunological reactivity with the CAPRIN-1 protein;

(G) an antibody or a fragment thereof comprising a heavy-chain variable region comprising complementarity determining regions of SEQ ID NOs: 182, 183, and 184 (CDR1, CDR2, and CDR3, respectively) and a light-chain variable region comprising complementarity determining regions of SEQ ID NOs: 185, 186, and 187 (CDR1, CDR2, and CDR3, respectively), and having an immunological reactivity with the CAPRIN-1 protein;

(H) an antibody or a fragment thereof comprising a heavy-chain variable region comprising complementarity determining regions of SEQ ID NOs: 188, 189, and 190 (CDR1, CDR2, and CDR3, respectively) and a light-chain variable region comprising complementarity determining regions of SEQ ID NOs: 191, 192, and 193 (CDR1, CDR2, and CDR3, respectively), and having an immunological reactivity with the CAPRIN-1 protein;

(I) an antibody or a fragment thereof comprising a heavy-chain variable region comprising complementarity determining regions of SEQ ID NOs: 146, 147, and 148 (CDR1, CDR2, and CDR3, respectively) and a light-chain variable region comprising complementarity determining regions of SEQ ID NOs: 149, 150, and 151 (CDR1, CDR2, and CDR3, respectively), and having an immunological reactivity with the CAPRIN-1 protein;

(J) an antibody or a fragment thereof comprising a heavy-chain variable region comprising complementarity determining regions of SEQ ID NOs: 272, 273, and 274 (CDR1, CDR2, and CDR3, respectively) and a light-chain variable region comprising complementarity determining regions of SEQ ID NOs: 275, 276, and 277 (CDR1, CDR2, and CDR3, respectively), and having an immunological reactivity with the CAPRIN-1 protein;

(K) an antibody or a fragment thereof comprising a heavy-chain variable region comprising complementarity determining regions of SEQ ID NOs: 290, 291, and 292 (CDR1, CDR2, and CDR3, respectively) and a light-chain variable region comprising complementarity determining regions of SEQ ID NOs: 293, 294, and 295 (CDR1, CDR2, and CDR3, respectively), and having an immunological reactivity with the CAPRIN-1 protein;

(L) an antibody or a fragment thereof comprising a heavy-chain variable region comprising complementarity determining regions of SEQ ID NOs: 301, 302, and 303 (CDR1, CDR2, and CDR3, respectively) and a light-chain variable region comprising complementarity determining regions of SEQ ID NOs: 305, 306, and 307 (CDR1, CDR2, and CDR3, respectively), and having an immunological reactivity with the CAPRIN-1 protein; and

(M) an antibody or a fragment thereof comprising a heavy-chain variable region comprising complementarity determining regions of SEQ ID NOs: 134, 135, and 136 (CDR1, CDR2, and CDR3, respectively) and a light-chain variable region comprising complementarity determining regions of SEQ ID NOs: 137, 138, and 139 (CDR1, CDR2, and CDR3, respectively), and having an immunological reactivity with the CAPRIN-1 protein.

5. The complex according to claim 1, wherein the antibody or the fragment thereof is any one of the following (a) to (al):

(a) an antibody or a fragment thereof comprising a heavy-chain variable region comprising the amino acid sequence of SEQ ID NO: 39 and a light-chain variable region comprising the amino acid sequence of SEQ ID NO: 43;

(b) an antibody or a fragment thereof comprising a heavy-chain variable region comprising the amino acid sequence of SEQ ID NO: 47 and a light-chain variable region comprising the amino acid sequence of SEQ ID NO: 51;

(c) an antibody or a fragment thereof comprising a heavy-chain variable region comprising the amino acid sequence of SEQ ID NO: 55 and a light-chain variable region comprising the amino acid sequence of SEQ ID NO: 59;

(d) an antibody or a fragment thereof comprising a heavy-chain variable region comprising the amino acid sequence of SEQ ID NO: 63 and a light-chain variable region comprising the amino acid sequence of SEQ ID NO: 67;

(e) an antibody or a fragment thereof comprising a heavy-chain variable region comprising the amino acid sequence of SEQ ID NO: 68 and a light-chain variable region comprising the amino acid sequence of SEQ ID NO: 69;

(f) an antibody or a fragment thereof comprising a heavy-chain variable region comprising the amino acid sequence of SEQ ID NO: 70 and a light-chain variable region comprising the amino acid sequence of SEQ ID NO: 71;

(g) an antibody or a fragment thereof comprising a heavy-chain variable region comprising the amino acid sequence of SEQ ID NO: 72 and a light-chain variable region comprising the amino acid sequence of SEQ ID NO: 73;

(h) an antibody or a fragment thereof comprising a heavy-chain variable region comprising the amino acid sequence of SEQ ID NO: 74 and a light-chain variable region comprising the amino acid sequence of SEQ ID NO: 75;

(i) an antibody or a fragment thereof comprising a heavy-chain variable region comprising the amino acid sequence of SEQ ID NO: 76 and a light-chain variable region comprising the amino acid sequence of SEQ ID NO: 77;

(j) an antibody or a fragment thereof comprising a heavy-chain variable region comprising the amino acid sequence of SEQ ID NO: 78 and a light-chain variable region comprising the amino acid sequence of SEQ ID NO: 79;

(k) an antibody or a fragment thereof comprising a heavy-chain variable region comprising the amino acid sequence of SEQ ID NO: 80 and a light-chain variable region comprising the amino acid sequence of SEQ ID NO: 81;

(l) an antibody or a fragment thereof comprising a heavy-chain variable region comprising the amino acid sequence of SEQ ID NO: 82 and a light-chain variable region comprising the amino acid sequence of SEQ ID NO: 83;

(m) an antibody or a fragment thereof comprising a heavy-chain variable region comprising the amino acid sequence of SEQ ID NO: 84 and a light-chain variable region comprising the amino acid sequence of SEQ ID NO: 85;

(n) an antibody or a fragment thereof comprising a heavy-chain variable region comprising the amino acid sequence of SEQ ID NO: 86 and a light-chain variable region comprising the amino acid sequence of SEQ ID NO: 87;

(o) an antibody or a fragment thereof comprising a heavy-chain variable region comprising the amino acid sequence of SEQ ID NO: 88 and a light-chain variable region comprising the amino acid sequence of SEQ ID NO: 89;

(p) an antibody or a fragment thereof comprising a heavy-chain variable region comprising the amino acid sequence of SEQ ID NO: 90 and a light-chain variable region comprising the amino acid sequence of SEQ ID NO: 91;

(q) an antibody or a fragment thereof comprising a heavy-chain variable region comprising the amino acid sequence of SEQ ID NO: 92 and a light-chain variable region comprising the amino acid sequence of SEQ ID NO: 93;

(r) an antibody or a fragment thereof comprising a heavy-chain variable region comprising the amino acid sequence of SEQ ID NO: 94 and a light-chain variable region comprising the amino acid sequence of SEQ ID NO: 95;

(s) an antibody or a fragment thereof comprising a heavy-chain variable region comprising the amino acid sequence of SEQ ID NO: 96 and a light-chain variable region comprising the amino acid sequence of SEQ ID NO: 97;

(t) an antibody or a fragment thereof comprising a heavy-chain variable region comprising the amino acid sequence of SEQ ID NO: 98 and a light-chain variable region comprising the amino acid sequence of SEQ ID NO: 99;

(u) an antibody or a fragment thereof comprising a heavy-chain variable region comprising the amino acid sequence of SEQ ID NO: 100 and a light-chain variable region comprising the amino acid sequence of SEQ ID NO: 101;

(v) an antibody or a fragment thereof comprising a heavy-chain variable region comprising the amino acid sequence of SEQ ID NO: 102 and a light-chain variable region comprising the amino acid sequence of SEQ ID NO: 103;

(w) an antibody or a fragment thereof comprising a heavy-chain variable region comprising the amino acid sequence of SEQ ID NO: 104 and a light-chain variable region comprising the amino acid sequence of SEQ ID NO: 105;

(x) an antibody or a fragment thereof comprising a heavy-chain variable region comprising the amino acid sequence of SEQ ID NO: 106 and a light-chain variable region comprising the amino acid sequence of SEQ ID NO: 107;

(y) an antibody or a fragment thereof comprising a heavy-chain variable region comprising the amino acid sequence of SEQ ID NO: 108 and a light-chain variable region comprising the amino acid sequence of SEQ ID NO: 109;

(z) an antibody or a fragment thereof comprising a heavy-chain variable region comprising the amino acid sequence of SEQ ID NO: 110 and a light-chain variable region comprising the amino acid sequence of SEQ ID NO: 111;

(aa) an antibody or a fragment thereof comprising a heavy-chain variable region comprising the amino acid sequence of SEQ ID NO: 112 and a light-chain variable region comprising the amino acid sequence of SEQ ID NO: 113;

(ab) an antibody or a fragment thereof comprising a heavy-chain variable region comprising the amino acid sequence of SEQ ID NO: 114 and a light-chain variable region comprising the amino acid sequence of SEQ ID NO: 115;

(ac) an antibody or a fragment thereof comprising a heavy-chain variable region comprising the amino acid sequence of SEQ ID NO: 116 and a light-chain variable region comprising the amino acid sequence of SEQ ID NO: 117;

(ad) an antibody or a fragment thereof comprising a heavy-chain variable region comprising the amino acid sequence of SEQ ID NO: 118 and a light-chain variable region comprising the amino acid sequence of SEQ ID NO: 119;

(ae) an antibody or a fragment thereof comprising a heavy-chain variable region comprising the amino acid sequence of SEQ ID NO: 120 and a light-chain variable region comprising the amino acid sequence of SEQ ID NO: 121;

(af) an antibody or a fragment thereof comprising a heavy-chain variable region comprising the amino acid sequence of SEQ ID NO: 122 and a light-chain variable region comprising the amino acid sequence of SEQ ID NO: 123;

(ag) an antibody or a fragment thereof comprising a heavy-chain variable region comprising the amino acid sequence of SEQ ID NO: 124 and a light-chain variable region comprising the amino acid sequence of SEQ ID NO: 125;

(ah) an antibody or a fragment thereof comprising a heavy-chain variable region comprising the amino acid sequence of SEQ ID NO: 126 and a light-chain variable region comprising the amino acid sequence of SEQ ID NO: 127;

(ai) an antibody or a fragment thereof comprising a heavy-chain variable region comprising the amino acid sequence of SEQ ID NO: 128 and a light-chain variable region comprising the amino acid sequence of SEQ ID NO: 129;

(aj) an antibody or a fragment thereof comprising a heavy-chain variable region comprising the amino acid sequence of SEQ ID NO: 130 and a light-chain variable region comprising the amino acid sequence of SEQ ID NO: 131;

(ak) an antibody or a fragment thereof comprising a heavy-chain variable region comprising the amino acid sequence of SEQ ID NO: 132 and a light-chain variable region comprising the amino acid sequence of SEQ ID NO: 133; and

(al) an antibody or a fragment thereof comprising a heavy-chain variable region comprising the amino acid sequence of SEQ ID NO: 300 and a light-chain variable region comprising the amino acid sequence of SEQ ID NO: 304.

6. The complex according to claim 1, wherein the antibody is a human antibody, a humanized antibody, or a chimeric antibody.

7. The complex according to claim 1, wherein the fragment is F(ab')$_2$, Fab', Fab, or scFv.

8. The complex according to claim 1, wherein the radioisotope is a radioisotope that radiates an $\alpha$ ray, a $\beta$ ray, or a $\gamma$ ray.

9. The complex according to claim 1, wherein the radioisotope is radium (Ra), thorium (Th), actinium (Ac), lutetium (Lu), indium (In), gallium (Ga), lead (Pb), copper (Cu), strontium (Sr), yttrium (Y), samarium (Sm), rhenium (Re), bismuth (Bi), technetium (Tc), or zirconium (Zr).

10. The complex according to claim 1, wherein the radioisotope is 223-Ra, 227-Th, 225-Ac, 177-Lu, 111-In, 67-Ga, 68-Ga, 212-Pb, 64-Cu, 67-Cu, 89-Sr, 90-Y, 153-Sm, 186-Re, 188-Re, 212-Bi, 213-Bi, 99m-Tc, or 89-Zr.

11. The complex according to claim 1, wherein the chelating agent is a bifunctional chelating agent.

12. The complex according to claim 11, wherein the bifunctional chelating agent is a cyclic or linear chelating agent.

13. The complex according to claim 12, wherein the cyclic chelating agent is selected from a group consisting of DOTA, NOTA, TETA, TCMC, DEPA, NETA, H$_2$macropa, Crown, PCTA, NODAGA, NODASA, Sar bicyclic chelator, MANOTA, and derivatives thereof.

14. The complex according to claim 12, wherein the linear chelating agent is selected from a group consisting of HOPO,

DTPA, MAG$_2$-GABA, Trisuccin, DFO, H$_2$dedpa, H$_4$octapa, HYNIC, HBED-CC, THP, and derivatives thereof.

15. A pharmaceutical composition for treatment, prevention, and/or diagnosis of a cancer, comprising, as an active ingredient, the complex according to any one of claims 1 to 14.

16. The pharmaceutical composition according to claim 15, wherein the cancer is a cancer expressing the CAPRIN-1 protein on a cell membrane surface.

17. The pharmaceutical composition according to claim 15, wherein the cancer is breast cancer, renal cancer, pancreatic cancer, colorectal cancer, lung cancer, brain tumor, stomach cancer, uterine cancer, ovarian cancer, prostate cancer, bladder cancer, esophageal cancer, leukemia, lymphoma, liver cancer, gallbladder cancer, bile duct cancer, sarcoma, mastocytoma, melanoma, adrenocortical carcinoma, Ewing's tumor, Hodgkin's lymphoma, mesothelioma, multiple myeloma, testicular cancer, thyroid cancer, head and neck cancer, or urothelial cancer.

18. A method of treating, preventing, and/or diagnosing a cancer, the method comprising administering the complex according to any one of claims 1 to 14 or the pharmaceutical composition according to claim 15 to a subject.

# EP 4 778 539 A1

## INTERNATIONAL SEARCH REPORT

| | International application No. |
|---|---|
| | **PCT/JP2024/032627** |

**A.    CLASSIFICATION OF SUBJECT MATTER**

*A61K 47/68*(2017.01)i; *A61K 39/395*(2006.01)i; *A61K 51/10*(2006.01)i; *A61P 35/00*(2006.01)i; *A61P 35/02*(2006.01)i; *C07K 16/18*(2006.01)i

FI:    A61K47/68 ZNA; A61P35/00; A61K51/10 100; A61K51/10 200; A61K39/395 N; A61K39/395 D; C07K16/18; A61P35/02

According to International Patent Classification (IPC) or to both national classification and IPC

**B.    FIELDS SEARCHED**

Minimum documentation searched (classification system followed by classification symbols)

A61K47/68; A61K39/395; A61K51/10; A61P35/00; A61P35/02; C07K16/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| Y | WO 2010/016526 A1 (TORAY INDUSTRIES, INC.) 11 February 2010 (2010-02-11) claims 1, 4-17, paragraphs [0054], [0090], examples 1-7 | 1-18 |
| Y | JP 2007-527403 A (BAYER SCHERING PHARMA AKTIENGESELLSCHAFT) 27 September 2007 (2007-09-27) claims 20-25, example 10 | 1-18 |
| Y | JP 2018-518476 A (PANACEA PHARMACEUTICALS INC.) 12 July 2018 (2018-07-12) examples 1-4 | 1-18 |
| Y | JP 2022-516170 A (ACTINIUM PHARMACEUTICALS, INC.) 24 February 2022 (2022-02-24) examples 1-4 | 1-18 |
| Y | WO 2011/096528 A1 (TORAY INDUSTRIES, INC.) 11 August 2011 (2011-08-11) claims 9-14 | 1-18 |

[✓] Further documents are listed in the continuation of Box C.          [✓] See patent family annex.

| | |
|---|---|
| *     Special categories of cited documents: | "T"   later document published after the international filing date or priority date and not in conflict with the application but cited to understand the principle or theory underlying the invention |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **18 November 2024** | **03 December 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2024/032627**

**C.  DOCUMENTS CONSIDERED TO BE RELEVANT**

| Category* | Citation of document, with indication, where appropriate, of the relevant passages | Relevant to claim No. |
|---|---|---|
| Y | WO 2015/020212 A1 (TORAY INDUSTRIES, INC.) 12 February 2015 (2015-02-12) claims 1-13 | 1-18 |
| Y | WO 2011/096519 A1 (TORAY INDUSTRIES, INC.) 11 August 2011 (2011-08-11) claim 4 | 1-18 |
| Y | WO 2013/125654 A1 (TORAY INDUSTRIES, INC.) 29 August 2013 (2013-08-29) claims 5-16 | 1-18 |
| Y | WO 2011/096517 A1 (TORAY INDUSTRIES, INC.) 11 August 2011 (2011-08-11) claims 7-8 | 1-18 |
| Y | WO 2011/096533 A1 (TORAY INDUSTRIES, INC.) 11 August 2011 (2011-08-11) claims 7-9 | 1-18 |
| Y | WO 2011/096534 A1 (TORAY INDUSTRIES, INC.) 11 August 2011 (2011-08-11) claims 7-9 | 1-18 |
| Y | WO 2013/018894 A1 (TORAY INDUSTRIES, INC.) 07 February 2013 (2013-02-07) claims 5-11 | 1-18 |
| Y | WO 2013/018892 A1 (TORAY INDUSTRIES, INC.) 07 February 2013 (2013-02-07) claim 1 | 1-18 |
| Y | WO 2013/018891 A1 (TORAY INDUSTRIES, INC.) 07 February 2013 (2013-02-07) claim 1 | 1-18 |
| Y | WO 2013/018889 A1 (TORAY INDUSTRIES, INC.) 07 February 2013 (2013-02-07) claim 1 | 1-18 |
| Y | WO 2013/018883 A1 (TORAY INDUSTRIES, INC.) 07 February 2013 (2013-02-07) claim 1 | 1-18 |
| Y | WO 2013/125636 A1 (TORAY INDUSTRIES, INC.) 29 August 2013 (2013-08-29) claim 5 | 1-18 |
| Y | WO 2013/125630 A1 (TORAY INDUSTRIES, INC.) 29 August 2013 (2013-08-29) claim 5 | 1-18 |

Form PCT/ISA/210 (second sheet) (July 2022)

**INTERNATIONAL SEARCH REPORT**

International application No.

**PCT/JP2024/032627**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2024/032627**

| Patent document cited in search report | Publication date (day/month/year) | Patent family member(s) | Publication date (day/month/year) |
|---|---|---|---|
| WO 2010/016526 A1 | 11 February 2010 | US 2011/0256144 A1 claims 1, 4-17, paragraphs [0058], [0094], examples 1-7 <br> EP 2322221 A1 <br> CN 102170907 A <br> KR 10-2011-0039475 A | |
| JP 2007-527403 A | 27 September 2007 | US 2005/0019845 A1 claims 20-25, example 10 <br> WO 2005/010048 A2 <br> EP 1648510 A1 <br> KR 10-2006-0054329 A <br> CN 1826138 A | |
| JP 2018-518476 A | 12 July 2018 | US 2016/0354499 A1 examples 1-4 <br> WO 2016/197102 A1 <br> EP 3302560 A1 <br> CN 107690334 A | |
| JP 2022-516170 A | 24 February 2022 | US 2022/0072167 A1 examples 1-4 <br> WO 2020/142583 A1 <br> EP 3906061 A1 | |
| WO 2011/096528 A1 | 11 August 2011 | US 2012/0301476 A1 claims 9-14 <br> EP 2532365 A1 <br> CN 102821788 A <br> KR 10-2012-0123500 A | |
| WO 2015/020212 A1 | 12 February 2015 | US 2016/0297889 A1 claims 1-13 <br> EP 3031826 A1 <br> CN 105452294 A <br> KR 10-2016-0038892 A | |
| WO 2011/096519 A1 | 11 August 2011 | US 2012/0321641 A1 claim 4 <br> EP 2532680 A1 <br> CN 102822199 A <br> KR 10-2012-0125327 A | |
| WO 2013/125654 A1 | 29 August 2013 | US 2015/0050283 A1 claims 5-16 <br> EP 2818483 A1 <br> CN 104114582 A <br> KR 10-2014-0130669 A | |
| WO 2011/096517 A1 | 11 August 2011 | US 2013/0071398 A1 claims 7-8 <br> EP 2532364 A1 <br> CN 102834113 A <br> KR 10-2012-0139719 A | |
| WO 2011/096533 A1 | 11 August 2011 | US 2013/0045210 A1 claims 7-9 <br> EP 2532366 A1 <br> CN 102821789 A <br> KR 10-2013-0033347 A | |

Form PCT/ISA/210 (patent family annex) (July 2022)

## INTERNATIONAL SEARCH REPORT
### Information on patent family members

International application No.

**PCT/JP2024/032627**

| Patent document cited in search report | | | Publication date (day/month/year) | Patent family member(s) | | | Publication date (day/month/year) |
|---|---|---|---|---|---|---|---|
| WO | 2011/096534 | A1 | 11 August 2011 | US | 2012/0301471 | A1 | |
| | | | | claims 7-9 | | | |
| | | | | EP | 2532743 | A1 | |
| | | | | CN | 102822335 | A | |
| | | | | KR | 10-2013-0004277 | A | |
| WO | 2013/018894 | A1 | 07 February 2013 | US | 2014/0186359 | A1 | |
| | | | | claims 5-11 | | | |
| | | | | EP | 2740796 | A1 | |
| | | | | CN | 103717740 | A | |
| | | | | KR | 10-2014-0054185 | A | |
| WO | 2013/018892 | A1 | 07 February 2013 | US | 2014/0199311 | A1 | |
| | | | | claim 1 | | | |
| | | | | EP | 2740793 | A1 | |
| | | | | CN | 103717738 | A | |
| | | | | KR | 10-2014-0054186 | A | |
| WO | 2013/018891 | A1 | 07 February 2013 | US | 2014/0154261 | A1 | |
| | | | | claim 1 | | | |
| | | | | EP | 2740794 | A1 | |
| | | | | CN | 103717739 | A | |
| | | | | KR | 10-2014-0054180 | A | |
| WO | 2013/018889 | A1 | 07 February 2013 | US | 2014/0178373 | A1 | |
| | | | | claim 1 | | | |
| | | | | EP | 2740795 | A1 | |
| | | | | CN | 103764825 | A | |
| | | | | KR | 10-2014-0054181 | A | |
| WO | 2013/018883 | A1 | 07 February 2013 | US | 2014/0193434 | A1 | |
| | | | | claim 1 | | | |
| | | | | EP | 2740798 | A1 | |
| | | | | CN | 103717737 | A | |
| | | | | KR | 10-2014-0054182 | A | |
| WO | 2013/125636 | A1 | 29 August 2013 | US | 2015/0004171 | A1 | |
| | | | | claim 5 | | | |
| | | | | EP | 2818482 | A1 | |
| | | | | CN | 104169303 | A | |
| | | | | KR | 10-2014-0130668 | A | |
| WO | 2013/125630 | A1 | 29 August 2013 | US | 2015/0044221 | A1 | |
| | | | | claim 5 | | | |
| | | | | EP | 2818481 | A1 | |
| | | | | CN | 104114581 | A | |
| | | | | KR | 10-2014-0130670 | A | |

Form PCT/ISA/210 (patent family annex) (July 2022)

## REFERENCES CITED IN THE DESCRIPTION

### Patent documents cited in the description

- WO 2010016526 A
- WO 2014012479 A
- EP 239400 A
- WO 9602576 A
- WO 9951743 A
- WO 2004063351 A
- WO 2011120135 A
- US 8388955 B
- WO 2011005481 A
- US 6737056 B
- WO 2005063351 A
- US 6602684 B
- EP 1914244 A
- US 7579170 B
- US 8642292 B
- WO 2011096517 A
- WO 2011096528 A
- WO 2011096519 A
- WO 2011096533 A
- WO 2011096534 A
- WO 2011096535 A
- WO 2013018886 A
- WO 2013018894 A
- WO 2013018892 A
- WO 2013018891 A
- WO 2013018889 A
- WO 2013018883 A
- WO 2013125636 A
- WO 2013125654 A
- WO 2013125630 A
- WO 2013125640 A
- WO 2013147169 A
- WO 2013147176 A
- WO 2015020212 A
- US 2022363768 A
- US 2019091354 A
- US 2017319721 A
- US 2015110817 A
- US 2010129315 A
- US 2008138899 A
- US 2023072421 A
- US 2022125960 A
- US 2022118123 A
- US 2017340759 A
- US 2019298865 A
- US 2011250137 A
- US 2011123444 A
- EP 1590005 A
- EP 2239274 A
- WO 2008046463 A
- WO 2008028530 A
- EP 1861127 A
- EP 1700608 A
- WO 2022162210 A
- WO 2022157094 A
- WO 2021160708 A
- WO 2012104225 A
- WO 2012069410 A
- WO 2011110490 A
- US 6183721 B
- US 5776894 A
- US 5753627 A
- US 5686410 A
- US 5650134 A
- US 2022401593 A
- US 2022378954 A
- EP 0515313 A
- WO 2022043557 A
- WO 2022043558 A
- EP 4034176 A
- WO 2023152671 A
- WO 2022219569 A
- WO 2022136317 A
- WO 2022043556 A
- US 2023321285 A
- US 2023338587 A
- US 2024050597 A
- US 2023357381 A
- US 2024197926 A
- US 2024197715 A
- US 2024252693 A
- WO 2024116053 A
- WO 2024121722 A
- EP 4279092 A
- US 2024108766 A
- US 2024156999 A
- US 2024245582 A
- WO 2023222557 A
- WO 2023159229 A
- WO 2004010957 A
- WO 2015057699 A
- WO 2017165851 A
- US 6214345 B
- WO 20070711968 A
- WO 2013173337 A

- WO 2015095755 A
- WO 2015123679 A
- WO 2018031690 A
- WO 200665533 A
- WO 2018160683 A

**Non-patent literature cited in the description**

- *J. Nucl. Med.*, 2005, vol. 46, 67S-75S
- *J. Nucl. Med.*, 2005, vol. 46 (1), 38S-47S
- **KARLIN** ; **ALTSCHUL**. *Proc. Natl. Acad. Sci. USA*, 1993, vol. 90, 5873-5877
- **ALTSCHUL et al.** *Nucleic Acids Res.*, 1997, vol. 25, 3389-3402
- **SATO K. et al.** *Cancer Research*, 1993, vol. 53, 851-856
- **KOHLER, G.** ; **MILSTEIN, C.** *Methods Enzymol.*, 1981, vol. 73, 3-46
- **CARL, A.K** ; **BORREBAECK** ; **JAMES** ; **W. LARRICK**. THERAPEUTIC MONOCLONAL ANTIBODIES. MACMILLAN PUBLISHERS LTD, 1990
- *Theranostics*, 2021, vol. 11 (13)
- *Molecules*, 2022, vol. 27, 3062
- **GREG T**. Hermanson Bioconjugate Techniques
- *Nature Biotechnology*, 2008, vol. 26, 925-932
- *Methods Mol Biol.*, 2020, vol. 2078, 113-129
- *MAbs.*, 2017, vol. 9 (6), 907-915
- **G. L. ELLMAN**. *Arch. Biochem. Biophys.*, 1959, vol. 82, 70
- *Biomedicines*, 2021, vol. 9, 1743
- *Anti-Cancer Drugs*, June 2017, vol. 28 (5), 489-502